# EUROPEAN PATENT SPECIFICATION

(11) **EP 1 916 298 B1**
(45) Date of publication and mention of the grant of the patent: **28.12.2011**
(21) Application number: 06766646.1
(22) Date of filing: 13.06.2006
(51) Int. Cl.: C12N 5/10, C12N 15/09, C12N 7/00, C12P 21/08

(54) **METHODS FOR PRODUCING MONOCLONAL ANTIBODIES**
VERFAHREN ZUR HERSTELLUNG VON MONOKLONALEN ANTIKÖRPERN
PROCEDES POUR LA PRODUCTION DES ANTICORPS MONOCLONAL

(30) Priority: 14.06.2005 JP 2005173851; 07.10.2005 JP 2005295451
(43) Date of publication of application: 30.04.2008
(73) Proprietor: Dnavec Corporation, Tsukuba-shi, Ibaraki 305-0856 (JP)
(72) Inventor: UEDA, Yasuji, Tsukuba-gun, Ibaraki 3002436 (JP); HARA, Hiroto c/o DNAVEC CORPORATION, Tsukuba-shi, Ibaraki 3050856 (JP); SHU, Tsugumine c/o DNAVEC CORPORATION, Tsukuba-shi, Ibaraki 3050856 (JP); HASEGAWA, Mamoru c/o DNAVEC CORPORATION, Tsukuba-shi, Ibaraki 3050856 (JP)
(74) Representative: Vossius & Partner
(86) International application number: PCT/JP2006/311835
(87) International publication number: WO 2006/134917

(56) References cited:
- WO-A-2004/052312
- WO-A1-01/72340
- WO-A1-2005/042737
- JP-A- 2000 253 876
- THE JAPANESE BIOCHEMICAL SOCIETY: 'Shinseikagaku Jikken Koza 12 Bunshi Men'ekigaku III- Kogen Kotai Hotai-' TOKYO KAGAKU DOJIN vol. 1ST EDITION, 05 February 1992, pages 1 - 2, AND 6 - 7, AND 25 - 27, XP003007676

### SEQUENCE LISTING

<110> DNAVEC CORPORATION
<120> METHOD FOR PRODUCTION OF ANTIBODY
<130> D4-A0402Y1P
<150> JP 2005-173851
   <151> 2005-06-14
<150> JP 2005-295451
   <151> 2005-10-07
<160> 12
<170>
<210> 1
   <211> 10
   <212> RNA
   <213> Artificial
<220>
   <223> S sequence (w= any one of a and c; v= any one of a, c, and g)
<400> 1
   cwuuvwcccu 10
<210> 2
   <211> 10
   <212> RNA
   <213> Artificial
<220>
   <223> S sequence
<400> 2
   cuuugacccu 10
<210> 3
   <211> 10
   <212> RNA
   <213> Artificial
<220>
   <223> S sequence
<400> 3
   cauucacccu 10
<210> 4
   <211> 10
   <212> RNA
   <213> Artificial
<220>
   <223> S sequence
<400> 4 10
   cuuucacccu
<210> 5
   <211> 10
   <212> DNA
   <213> Artificial
<220>
   <223> S sequence
<400> 5
   agggtcaaag 10
<210> 6
   <211> 10
   <212> DNA
   <213> Artificial
<220>
   <223> S sequence
<400> 6
   agggtgaatg 10
<210> 7
   <211> 10
   <212> DNA
   <213> Artificial
<220>
   <223> S sequence
<400> 7
   agggtgaaag 10
<210> 8
   <211> 9
   <212> RNA
   <213> Artificial
<220>
   <223> E sequence
<400> 8
   uuuuucuua 9
<210> 9
   <211> 9
   <212> DNA
   <213> Artificial
<220>
   <223> E sequence
<400> 9
   taagaaaaa 9
<210> 10
   <211> 16
   <212> PRT
   <213> Artificial
<220>
   <223> Human immunodeficiency virus type 1 (gp160 partial polypeptide)
<400> 10
   Thr Arg Lys Arg Ile Arg Ile Gln Arg Gly Pro Gly Arg Ala Phe Val
   1 5 10 15
<210> 11
   <211> 16
   <212> PRT
   <213> Artificial
<220>
   <223> Human immunodeficiency virus type 1 (gp160 partial polypeptide)
<400> 11
   Ser Glu Leu Tyr Lys Tyr Lys Val Val Lys Ile Glu Pro Leu Gly Val
   1               5                   10                  15
<210> 12
   <211> 17
   <212> PRT
   <213> Artificial
<220>
   <223> Human immunodeficiency virus type 1 (gp160 partial polypeptide)
<400> 12
   Pro Arg Gly Pro Asp Arg Pro Glu Gly Ile Glu Glu Glu Gly Gly Glu
   1               5                   10                  15
   Arg

## Description

### Technical Field

The present invention relates to methods for producing antibodies and antibody-producing cells.

### Background Art

To reveal the association between the quantity of protein expression and the cellular function or cellular state is one of the biggest hurdles of the post-genomic era. However, it is very difficult to associate proteins with their function through expression analyses at the gene level alone. Therefore, detection and identification of proteins or peptide chains are essential. To analyze a protein, various techniques, such as amino acid sequence and composition analyses, secondary or tertiary structure analyses, and molecular weight identification, have been developed. However, methods using antibodies remain the standard techniques for identifying proteins (Michael Steward, Chapter 27. Immunological Techniques, pp. 417-434, in Immunology, 6th edition (Ivan M. Roitt, Jonathan Brostoff, David K. Male, Editors), 2001, C.V. Mosby). Of course, antibodies are not only used for a variety of protein detections and identification methods, such as Western blotting, immunohistochemistry, flow cytometry, immunoprecipitation, and immunoaffinity column methods, but are also widely used in practical applications, such as missile therapy against cancer cells, vector targeting, and inhibition of cellular function.

High levels of binding specificity and binding performances of antibodies are critical to their utilities, the value of which appears to have markedly increased by the invention of methods for producing monoclonal antibodies. However, the production of antibodies having a sufficient affinity to a target epitope is often very difficult for some proteins due to their shape and property. To produce antibodies, animals are often immunized with purified proteins, synthetic peptides, isolated protein-expressing cells, isolated tissues, and the like. Meanwhile, to produce antibodies to a protein/peptide chain based on specific gene information, there are methods in which an expression plasmid carrying said gene is directly inoculated into an animal tissue, and methods in which the gene is expressed in cultured cells and then an animal is immunized with a crude fraction of the cells. However, the expression level is often insufficient to obtain desired antibodies.
[Non-Patent Document 1] Michael Steward, Chapter 27. Immunological Techniques, pp. 417-434, in Immunology, 6th edition (Ivan M. Roitt, Jonathan Brostoff, David K. Male, Editors), 2001, C.V. Mosby.

### Disclosure of the Invention

### Problems to be Solved by the Invention

The present invention provides methods for producing antibodies and antibody-producing cells. Moreover, the present invention provides methods for producing antigen compositions for producing antibodies.

### Means for Solving the Problems

Minus-strand RNA Viruses have a minus-strand RNA as their genome. When they infect cells, the genomic RNA is amplified in the cell, and carried genes are expressed at high level (Yonemitsu Y. et al., Nat Biotechnol, 2000, 18(9):970-3). The present inventors thought of using minus-strand RNA viruses to produce antibodies, taking advantage of the above merit of minus-strand RNA viruses. Moreover, minus-strand RNA viruses have been reported to increase the immune activity of hosts after infection by inducing the secretion of a number of cytokines (Strahle et al. J. Virol. 77(14): 7903-7913 (2003), Tsutsumi et al. Pediatr. Infect. Dis. J. 20(10): 997-1001 (2001)). Thus, this point is also expected to be advantageous in producing antibodies. Therefore, the present inventors produced recombinant minus-strand RNA viruses expressing polypeptides to be used as antigens, and immunized animals with the viruses to produce antibodies.

The minus-strand RNA virus as it is, or a lysate of cells infected with the virus, was inoculated into animals by multiple administration routes such as nasal administration, intramuscular injection, direct splenic injection, subcutaneous injection in the lower back, and injection in the foot pad. Examination of antibody production revealed detectable antibodies in the blood regardless of administration route. In particular, the antibody titer was markedly increased with the nasal administration. The level of antibody production was further increased through use of intraperitoneal booster administration. Furthermore, hybridomas were produced from spleen cells of the immunized mice, and monoclonal antibodies were successfully produced. It is considered that the antibody production was efficiently induced by the immunoactivation effect of the minus-strand RNA viral vector and the high level expression of the antigen polypeptide.

Conventionally, subcutaneous administration, intracutaneous administration, intraperitoneal administration, administration to the foot pad, and the like have been employed for immunizing animals with antigen polypeptides, and intramuscular injection has been employed when using antigen polypeptide-expressing plasmids. Minus-strand RNA viral vectors, particularly Sendai virus vectors, provide nasal administration as a new administration method for immunization, in addition to the above-mentioned administrations. Moreover, in conventional methods of intramuscularly injecting an antigen polypeptide-expressing plasmid, it is difficult to reliably induce the immunity, and it is known that the immunity induced by the methods does not in many cases attain a sufficient level. Since Sendai virus vectors efficiently infect many cells and express foreign genes at high levels, they can reliably and readily induce antibody production. Furthermore, methods for producing antibodies using Sendai virus vectors are also advantageous in that it is possible to use improved vectors whose safety has been enhanced from the viewpoint of bio safety, which, for example, do not produce infectious particles in infected cells. (WO00/70055, WO00/70070; Li, H.-O. et al., J. Virol. 74(14) 6564-6569 (2000)).

As described above, the present invention demonstrates that antibodies against an antigen polypeptide can be efficiently produced by inoculating a minus-strand RNA viral vector expressing the antigen polypeptide. The vector inoculation site is not limited, and boosting is also effective. Immunization is possible not only by direct inoculation of a minus-strand RNA viral vector, but also using cultured cells infected with the vector. The produced cells can be fused for immunization, and in addition, desired antigen polypeptides can be purified from the cells and used for detecting antibodies by ELISA or Western blotting, screening for monoclonal antibody-producing hybridomas, and the like. Antibodies obtained by the methods of the present invention are expected to be applicable to a wide range of fields, including detection and isolation of biomolecules and antibody therapy. Thus, the present invention relates to methods for producing antibodies and antibody-producing cells using minus-strand RNA viruses, and the like. More specifically, the present invention relates to the invention described in each claim. Inventions composed of a combination of one or more inventions set forth in claims that cite the same claim are intended by the invention set forth in said claim. Specifically, the present invention provides:
[1] a method for producing an antibody or an antibody-producing cell, which comprises the steps of:
   (a) inoculating a non-human animal with a minus-strand RNA viral vector carrying a nucleic acid which encodes a foreign polypeptide to be used as an antigen, a nucleic acid producing the viral vector, a cell into which the vector or the nucleic acid producing the vector has been introduced, or a lysate of the cell; and
   (b) collecting an antibody or an antibody-producing cell from the animal;
[2] the method of [1], wherein the inoculation of step (a) is performed by an administration route selected from the group consisting of intramuscular injection, subcutaneous administration, nasal administration, palm or foot pad intracutaneous administration, splenic administration, and intraperitoneal administration;
[3] the method of [1] or [2], further comprising the step of boosting by inoculating with said minus-strand RNA viral, vector, said nucleic acid producing the viral vector, said cell into which the vector or the nucleic acid producing the vector has been introduced, said lysate of the cell, or an antigen purified from the lysate of the cell;
[4] the method of any one of [1] to [3], further comprising the step of boosting with said polypeptide or a polypeptide comprising a fragment thereof;
[5] the method of any one of [1] to [4], further comprising the step of contacting the collected antibody with said antigen to select an antibody which binds to the-antigen;
[6] the method of any one of [1] to [4], further comprising the step of fusing the collected antibody-producing cell with a Myeloma to prepare a hybridoma;
[7] the method of [6], further comprising the step of contacting an antibody produced by the hybridoma with said antigen to select a hybridoma producing an antibody that binds to the antigen;
[8] the method of [6] or [7], further comprising the step of collecting a monoclonal antibody produced by the hybridoma;
[9] the method of any one of [1] to [8], wherein the minus-strand RNA virus is replication-defective;
[10] the method of any one of [1] to [9], wherein the minus-strand RNA virus is a paramyxovirus;
[11] the method of [10], wherein the paramyxovirus is at least F gene-defective;
[12] the method of [10] or [11], wherein the paramyxovirus is Sendai virus;
[13] the method of any one of [1] to [12], further comprising the steps of:
   allowing a solution comprising the collected antibody to coexist with said minus-strand RNA viral vector that does not encode said foreign polypeptide, a cell into which the viral vector has been introduced, a lysate of the cell, or a viral protein of the virus; and
   selecting an antibody that does not bind thereto;
[14] the method of any one of [1] to [12], further comprising the steps of:
   allowing a solution comprising an antibody produced by the hybridoma to coexist with a minus-strand RNA viral vector that does not encode said foreign polypeptide, a cell into which such a viral vector has been introduced, a lysate of the cell, or a viral protein of the virus; and
   selecting a hybridoma producing an antibody that does not bind thereto;
[15] the method of any one of [1] to [14], further comprising the step of administering a Th2 cytokine or an active partial peptide thereof, or a vector encoding the same;
[16] the method of [15], wherein the cytokine or active partial peptide thereof is encoded by the minus-strand RNA viral vector which encodes the foreign polypeptide to be used as an antigen; and
[17] the method of [15] or [16], wherein the Th2 cytokine is selected from the group consisting of IL-4, IL-10, IL-13.

### Effects of the Invention

The methods of the present invention are capable of efficiently producing antibodies using a minus-strand RNA virus expressing a desired antigen polypeptide. The purification of the antigen peptide is not necessary. Antibodies produced in accordance with the methods of the present invention can be used not only in various protein detections and identification methods, such as Western blotting, immunohistochemistry, flow cytometry, immunoprecipitation, and immunoaffinity column methods, but also in clinical fields, such as missile therapy against cancer cells, vector targeting, and inhibition of cellular function.

### Brief Description of the Drawings

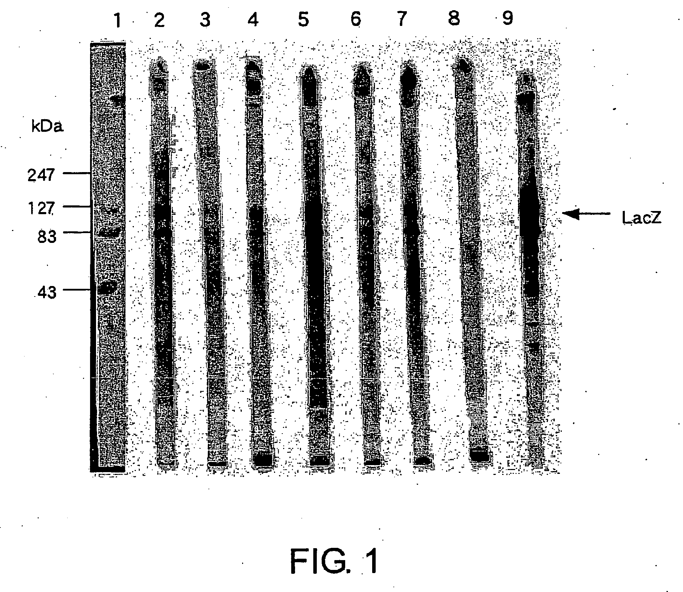

Fig. 1 depicts the production of anti-LacZ antibodies following the administration of a minus-strand RNA viral vector carrying a LacZ gene. The antibodies were detected with a commercially-available purified LacZ, using the plasma of mice on the fifth week after priming by intramuscular injection.

### [Description of each lane]

1. Molecular weight marker;
2. SeV-LacZ inoculated mouse #1;
3. SeV-LacZ inoculated mouse #2;
4. SeV-LacZ inoculated mouse #3;
5. SeV-LacZ inoculated mouse #4;
6. SeV-LacZ inoculated mouse #5;
7. SeV-LacZ inoculated mouse #6;
8. Nonimmunized mouse;
9. Commercially-available anti-LacZ antibody (1/5000 dilution).

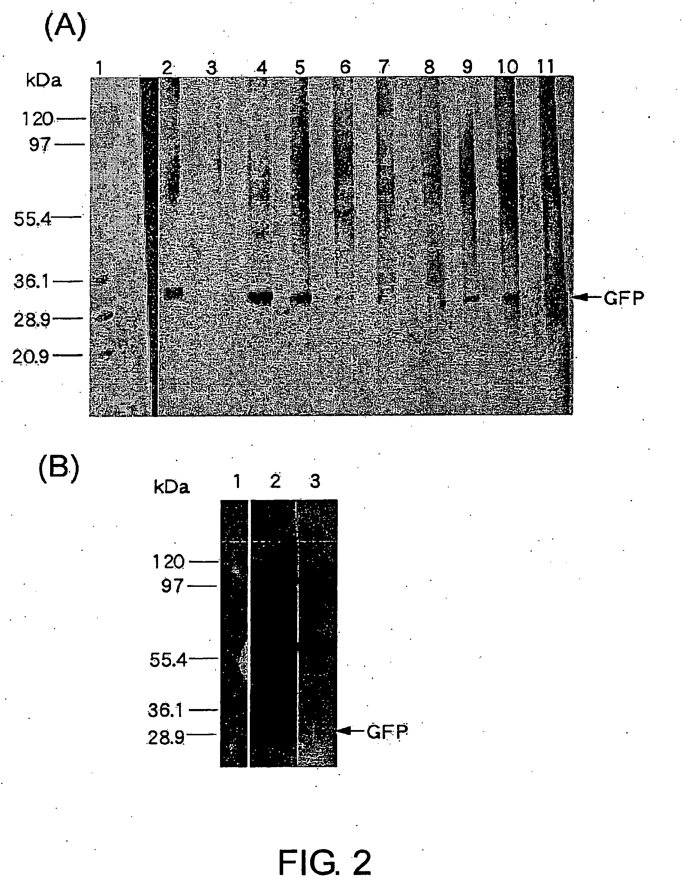

Fig. 2 depicts the production of anti-GFP antibodies following the administration of a minus-strand RNA viral vector carrying a GFP gene.

A: Priming was performed by nasal administration of the viral vector, intramuscular injection of the viral vector, direct splenic injection of the viral vector, or injection of the viral vector in the foot pad. After two weeks, boosting was performed by the intraperitoneal administration of the same viral vector. After four weeks of the priming, anti-GFP antibodies in the mouse plasma were detected by Western blotting.

### [Description of each lane]

1. Molecular weight marker;
2. Commercially-available anti-GFP antibody;
3. Nonimmunized mouse;
4. Priming by nasal administration/Boosting with Sendai virus vector;
5. Priming by nasal administration/Boosting with PBS (Control);
6. Priming by intramuscular injection /Boosting with Sendai virus vector;
7. Priming by intramuscular injection/Boosting with PBS (Control);
8. Priming by splenic injection/Boosting with Sendai virus vector;
9. Priming by splenic injection/Boosting with PBS (Control);
10. Priming by foot pad injection/Boosting with Sendai virus vector;
11. Priming by foot pad injection/Boosting with PBS (Control).

B: Priming was performed by the intracutaneous or intraperitoneal administration of a Sendai virus vector. After 16 days, boosting was performed by intraperitoneal administration of the Sendai virus vector (1x10⁷ CIU/head). After 19 days from the priming, anti-GFP antibodies in the mouse plasma were detected by Western blotting. The lysate of LLC-MK₂ cells infected with the GFP gene-carrying Sendai virus vector was immobilized onto a membrane.

### [Description of each lane]

1. Molecular weight marker;
2. Priming by subcutaneous administration;
3. Priming by intraperitoneal administration.

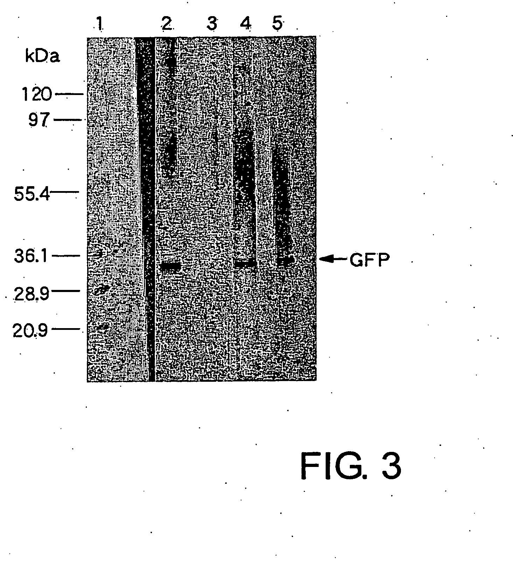

Fig. 3 depicts the production of antibodies following intraperitoneal administration of the lysate of cells infected with the minus-strand RNA viral vector. After two weeks of priming by intraperitoneal administration of the lysate of cells infected with the GFP gene-carrying minus-strand RNA viral vector, boosting was performed by intraperitoneal administration of the same vector. After four weeks of the priming, anti-GFP antibodies in the mouse plasma were detected by Western blotting.

### [Description of each lane]

1. Molecular weight marker;
2. Commercially-available anti-GFP antibody;
3. Nonimmunized mouse;
4. Priming by intraperitoneal administration of the lysate/Boosting with Sendai virus vector;
5. Priming by intraperitoneal administration of the lysate/Boosting with PBS (Control).

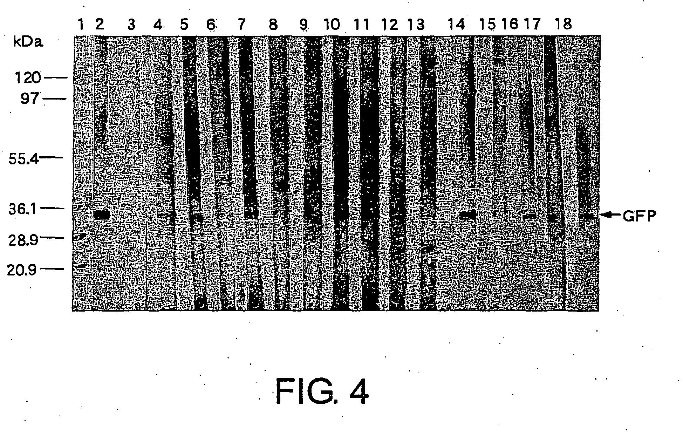

Fig. 4 depicts the effects of boosting by intraperitoneal administration of the minus-strand RNA viral vector, on the production of antibodies. Priming was performed by nasal administration, intramuscular injection, direct splenic injection, or foot pad injection of a GFP gene-carrying Sendai virus vector, or by intraperitoneal administration of the lysate of cells infected with the Sendai virus vector. After two weeks, boosting was performed by intraperitoneal administration of the lysate of cells infected with the Sendai virus vector. After four weeks of the priming, anti-GFP antibodies in the mouse plasma were detected by Western blotting. In control animals, the boosting operation after two weeks was performed with DPBS(-).

### [Description of each lane]

1. Molecular weight marker;
2. Commercially-available anti-GFP antibody;
3. Nonimmunized mouse;
4. Priming by nasal administration of the vector/Boosting with lysate-1;
5. Priming by nasal administration of the vector /Boosting with lysate - 2;
6. Priming by intramuscular injection of the vector /Boosting with lysate-1;
7. Priming by intramuscular injection of the vector /Boosting with lysate - 2;
8. Priming by splenic injection of the vector /Boosting with lysate -1;
9. Priming by splenic injection of the vector /Boosting with lysate - 2;
10. Priming by foot pad injection of the vector /Boosting with lysate-1;
11. Priming by foot pad injection of the vector /Boosting with lysate-2;
12. Priming by intraperitoneal administration of the lysate /Boosting with lysate-1;
13. Priming by intraperitoneal administration of the lysate /Boosting with lysate-2;
14. Priming by nasal administration of the vector /Boosting with PBS (Control);
15. Priming by intramuscular injection of the vector /Boosting with PBS (Control);
16. Priming by splenic injection of the vector /Boosting with PBS (Control);
17. Priming by foot pad injection of the vector /Boosting with PBS (Control);
18. Priming by intraperitoneal administration of the lysate /Boosting with PBS (Control).

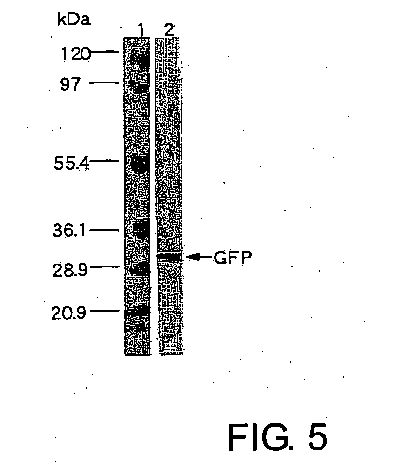

Fig. 5 depicts the production of monoclonal antibodies following the administration of the minus-strand RNA viral vector. Using an obtained hybridoma supernatant, GFP was detected on a Western blot membrane on which soluble proteins of LLC-MK₂ cells infected with the GFP gene-carrying Sendai virus vector are immobilized.

### [Description of each lane]

1. Molecular weight marker;
2. Immobilized with soluble proteins of LLC-MK₂ cells infected with GFP gene-carrying Sendai virus vector; probed using hybridoma supernatant

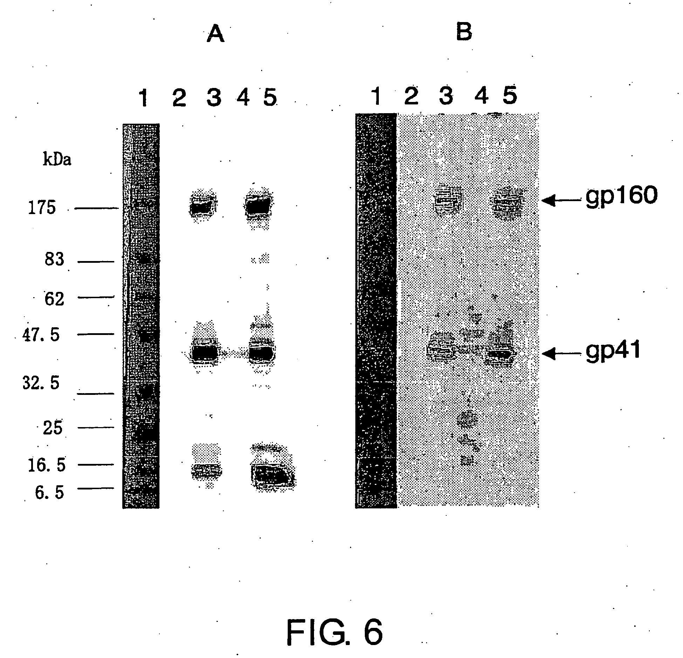

Fig. 6 depicts the result of Western blot analysis using an anti-gp160 monoclonal antibody-producing hybridoma supernatant (#6-76). Hybridomas were produced from splenocytes of a mouse inoculated with the HTV-1 gp160 gene-carrying minus-strand RNA viral vector, and a hybridoma which produced anti-gp160 monoclonal antibodies was selected. Using this hybridoma supernatant, the gp160 protein in the lysate of LLC-MK₂ cells infected with the SeV18+GP160/ΔF vector was detected by Western blotting (Panel A). Western blotting was performed in the same manner using commercially-available anti-gp160 antibodies (ICN) as positive control (Panel B).

### [Description of each lane]

1. Molecular weight marker;
2. Non-infected LLC-MK₂ cell lysate;
3. SeV18+GP160/ΔR-infected LLC-MK₂ cell lysate;
4. Non-infected BHK-21 cell lysate;
5. SeV18+GP160/AF-infected BHK-21 cell lysate.

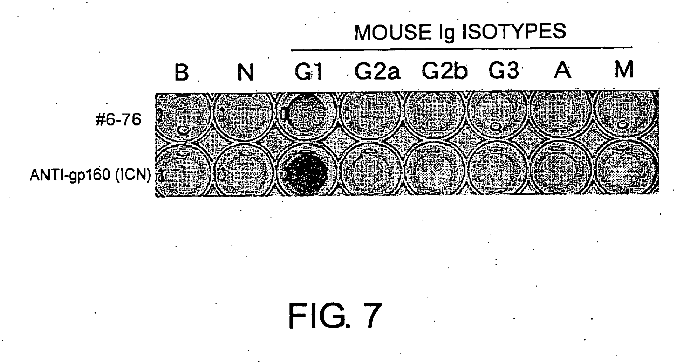

Fig. 7 demonstrates the determination of the subclass of an anti-gp160 antibody produced by the hybridoma #6-76. The subclass of anti-gp160 antibody produced by the hybridoma #6-76 was determined using a commercially available isotyping kit (Mouse Immunoglobulin Screening/Isotyping Kit (Genzyme Corporation)). The subclass-specific antibodies used were shown on the top.
G1: IgG₁, G2a: IgG₂ₐ, G2b: IgG_{2b}, G3: IgG₃, A: IgA, M: IgM, B: buffer blank, N: nonimmunized serum (negative control)

Fig: 8 demonstrates that priming with a Sendai virus vector followed by boosting with synthetic peptides enables the production of a hybridoma producing a monoclonal antibody which recognizes HIV-1 gp160. Using a culture supernatant of a hybridoma C1-182 that was positive in the primary screening by ELISA, the gp160 protein in a membrane fraction of BHK-21 cells infected with SeV18+GP160/ΔF vector was detected by Western blotting. The membrane fraction was prepared using a commercially available kit (Calbiochem, Cat. No. 539790) according to the instructions. The migration positions of gp160 and gp41 are shown by the arrows on the right.

### [Description of each lane]

1. Molecular weight marker;
2. Non-infected BHK-21 cell membrane fraction;
3. SeV18+GFP/ΔF-infected BHK-21 cell membrane fraction;
4. SeV18+GP160/ΔF-infected BHK-21 cell membrane fraction.

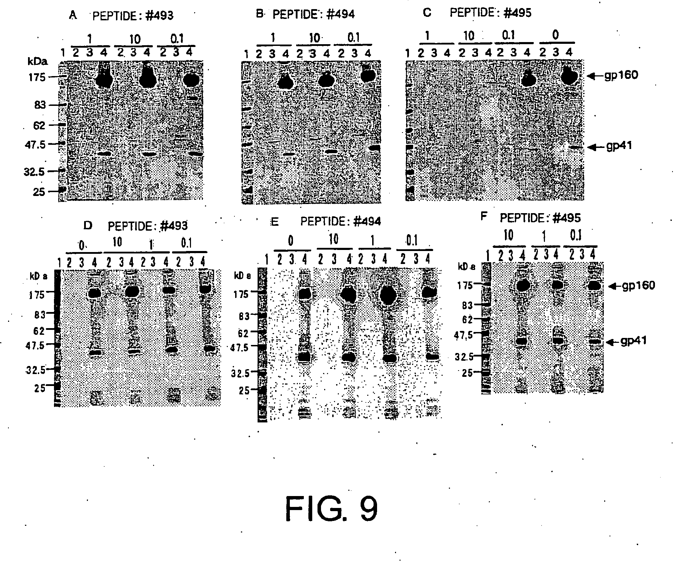

Fig. 9 demonstrates that an epitope in the gp160 protein recognized by the anti-gp160 antibody-producing hybridoma C1-182, which was produced by boosting using synthetic peptides, was detected by mixing a culture supernatant with the peptides used for the boosting and then performing Western blotting. When the C1-182 supernatant was used, only the case where peptide #495 was mixed showed the inhibition of antibody reaction in a peptide concentration-dependent manner, demonstrating that the sequence of peptide #495 was the epitope recognized by the C1-182 antibody. The numbers 0, 0.1, 1, and 10 in the panel denote the concentration of the mixed peptide. The unit is µg/ml. The migration positions of gp160 and gp41 are shown by the arrows on the right.

### [Description of each panel]

A. to C. Hybridoma C1-182 supernatant was used.
D. to F. Hybridoma #6-76 supernatant was fused.
A. and D. Peptide #493 was mixed.
B. and E. Peptide #494 was mixed.
C. and F. Peptide #495 was mixed.

### [Description of each lane]

1. Molecular weight maker;
2. Non-infected BHK-21 cell membrane fraction;
3. SeV18+GFP/ΔF-infected BHK-21 cell membrane fraction;
4. SeV18+GP160/ΔF-infected BHK-21 cell membrane fraction.

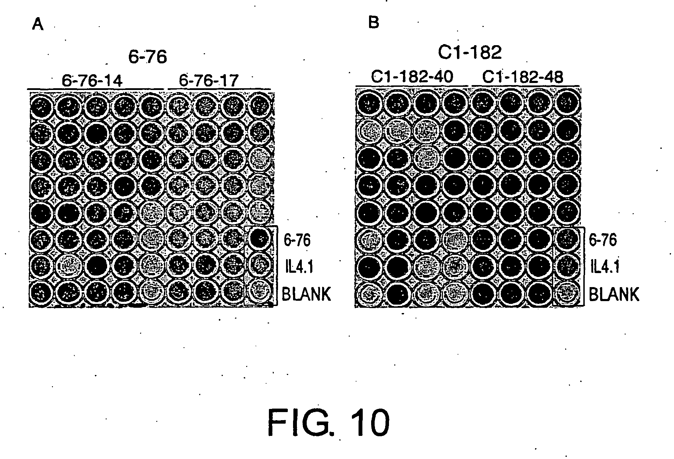

Fig. 10 depicts the purification of the anti-gp160 antibody-producing hybridoma #6-76 obtained in Example 6 and the anti-gp160 antibody-producing hybridoma C1-182 obtained by boosting using the synthetic peptides in Example 7, by cell cloning. The figure shows the result of ELISA on supernatants of colonies recloned through diluting and seeding #6-76-14 and #6-76-17, which were obtained in the primary cloning of hybridoma #6-76, and Cm-182-40 and C1-182-48, which were obtained in the primary cloning of hybridoma C1-182. The cloning was successful except for C1-182-40. For a positive control, the supernatant of uncloned hybridoma #6-76 and the anti-Sendai virus HN protein monoclonal antibody IL4.1 were used.

### [Description of each panel]

A. Supernatants of secondary cloned colonies derived from hybridoma #6-76 were used.
B. Supernatants of secondary cloned colonies derived from hybridoma C1-182 were used.

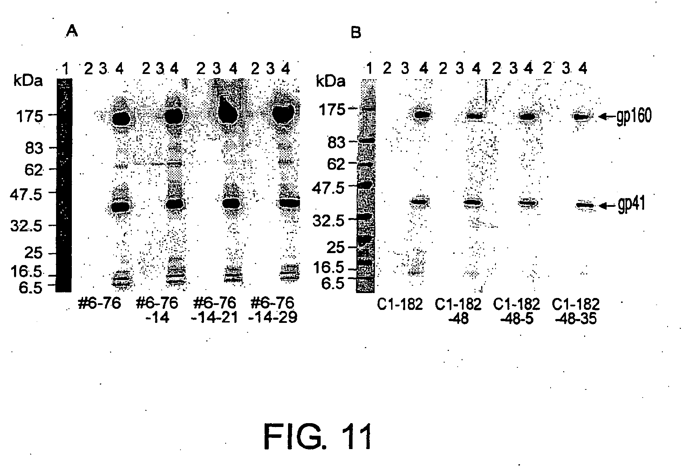

Fig. 11 depicts the purification of hybridoma #6-76 obtained in Example 6 and hybridoma C1-182 obtained in Example 7, by cell cloning. Western blotting was performed in the same manner as in Example 7 using #6-76-14-21 and #6-76-14-29, which were obtained by recloning #6-76-14 yielded from the primary cloning of hybridoma #6-76; and C1-182-48-5 and C1-182-48-35, which were obtained by recloning C1-182-48 yielded from the primary cloning of hybridoma C1-182. It was shown that the secondary cloned hybridomas also produced the same anti-gp 160 antibodies as prior to the cloning.

### [Description of each panel]

A. Supernatants of hybridoma #6-76, primary cloned colony #6-76-14, and secondary cloned colonies #6-76-14-21 and #6-76-14-29 were used.
B. Supernatants of hybridoma C1-182, primary cloned colony C1-182-48, and secondary cloned colonies C1-182-48-5 and C1-182-48-35 were used.

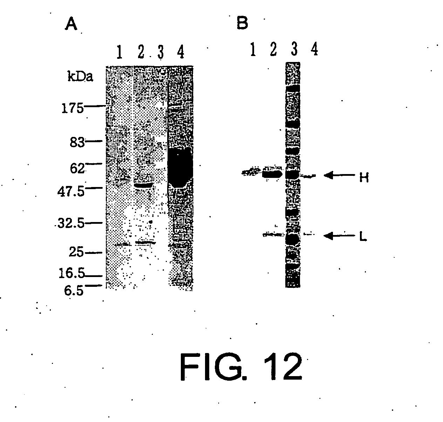

fig. 12 depicts the results of the purification of antibody proteins in the culture supernatant of #6-76-14-29, which was obtained by cloning twice hybridoma #6-76 obtained in Example 6, using a column immobilized with immunoglobulin-binding protein L (ImmunoPure Immobilized Protein L from Pierce; Cat. No. 20510). It was shown that the secondary cloned hybridoma also produced the same anti-gp160 antibodies as prior to the cloning. The migration positions of IgG H chain and L chain are shown by the arrows on the right.

### [Description of each panel]

A. Sypro Orange staining
B. Anti-mouse IgG (H+L) antibody (HRP-conjugate) staining

### [Description of each lane]

1. Anti-ovalbumin mouse monoclonal antibody;
2. Purified #6-76-14-29 supernatant;
3. Molecular weight marker;
4. Unpurified #6-76-14-29 supernatant.

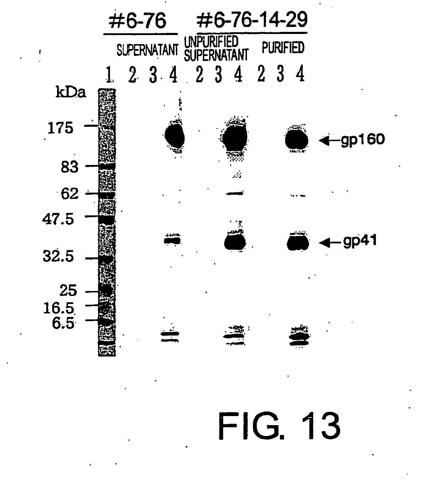

Fig. 13 depicts the results of Western blotting performed in the same manner as in Example 7, using antibody proteins purified from the culture supernatant of the secondary cloned hybridoma #6-76-14-29. Unpurified culture supernatants of uncloned #6-76 and #6-76-14-29 were compared with the purified antibody. It was shown that the purified antibody recognized gp160 and gp41 with the same specificity as prior to the purification.

### [Description of each lane]

1. Molecular weight marker;
2. Non-infected BHK-21 cell membrane fraction;
3. SeV18+GFP/ΔF-infected BHK-21 cell membrane fraction;
4. SeV18+GP160/ΔF-infected BHK-21 cell membrane fraction.

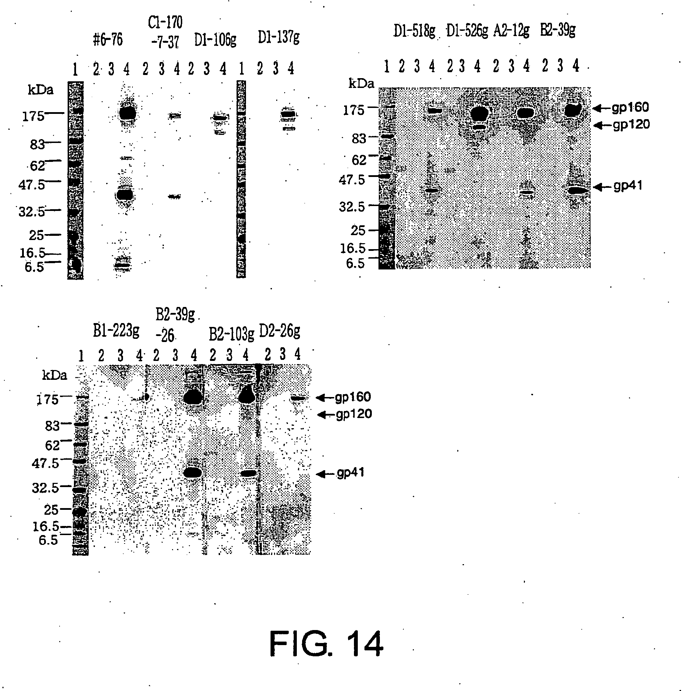

Fig. 14 depicts the results of Western blotting using the culture supernatants of anti-gp160 antibody-producing hybridomas obtained in Example 10. Two types of hybridomas, hybridomas recognizing the gp41 moiety in gp160 and hybridomas recognizing gp120 moiety, were obtained. The migration positions of gp160, gp120, and gp41 were shown by the arrows on the right.

### [Description of each lane]

1. Molecular weight marker;
2. Non-infected BHK-21 cell membrane fraction;
3. SeV18+GFP/ΔF-infected BHK-21 cell membrane fraction;
4. SeV18+GP160/ΔF-infected BHK-21 cell membrane fraction.

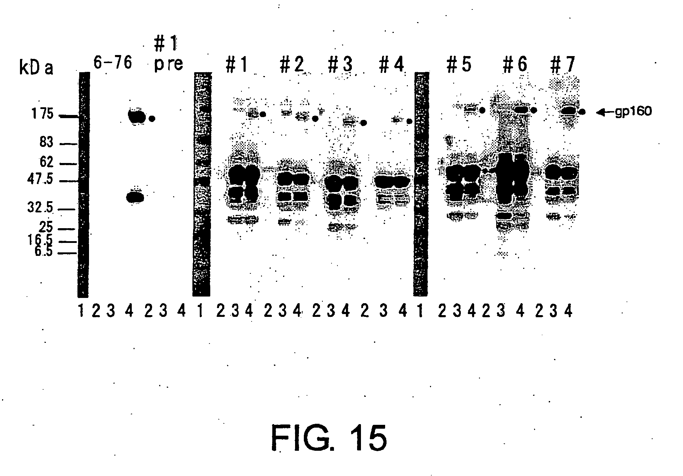

Fig. 15 demonstrates that anti-gp 160 antibodies are induced in mouse plasma subsequent to immunization with a Sendai virus vector carrying both a mouse IL10 gene and a gp160 gene. Seven mice were used. The blood was harvested from the orbit on day 56 after priming. The culture supernatant of hybridoma #6-76 was used as a positive control, and the mouse plasma prior to the immunization (#1 pre) was used as a negative control. The migration position of gp160 was shown by the arrow on the right and the black dots.

### [Description of each lane]

1. Molecular weight marker;
2. Non-infected BHK-21 cell membrane fraction;
3. SeV18+GFP/ΔF-infected BHK-21 cell membrane fraction;
4. SeV18+GP160/ΔF-infected BHK-21 cell membrane fraction.

### Best Mode for Carrying Out the Invention

The present invention relates to methods for producing antibodies and antibody-producing cells, which include the steps of: inoculating a non-human animal with a minus-strand RNA viral vector carrying a nucleic acid which encodes a foreign polypeptide to be used as an antigen, nucleic acids producing the viral vector, a cell into which the vector or the nucleic acids producing the vector were introduced, or a lysate of the cells; and collecting antibodies or antibody-producing cells from the animal. A minus-strand RNA virus refers to viruses that contains a minus strand (an antisense strand complementary to a sense strand encoding viral proteins) RNA as the genome. The minus-strand RNA virus is also referred to as negative strand RNA virus. In the context of the present invention, preferred minus-strand RNA viruses particularly include single-stranded minus-strand RNA viruses (also referred to as non-segmented minus-strand RNA viruses). The term "single-strand negative strand RNA virus" refers to viruses having a single-stranded negative strand [i.e., a minus strand] RNA as the genome. Such viruses include viruses belonging to Paramyxoviridae (including the genera Paramyxovirus, Morbillivirus, Rubulavirus, and Pneumovirus), Rhabdoviridae (including the genera Vesiculovirus, Lyssavirus, and Ephemerovirus), Filoviridae, and the like.

Minus-strand RNA viral vectors used in the present invention may be transmissible, or may be defective, nontransmissible vectors. "Transmissible" means that when a viral vector inflects a host cell, the virus is replicated in the cell to produce infectious virions. Examples of defective vectors include vectors lacking at least one of genes that encode proteins constituting envelopes. Specific examples include vectors lacking at least one of genes encoding envelope-constituting proteins such as F, H, HN, G, M, and M1, depending on the type of virus (WO00/70055, WO00/70070; Li, H.-O. et al., J. Virol. 74(14) 6564-6569 (2000)).

Specific examples of particularly preferred minus-strand RNA viruses suitable for use in the context of the present invention include, for example, Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3 belonging to Paramyxoviridae; influenza virus belonging to Orthomyxoviridae; and vesicular stomatitis virus and rabies virus belonging to Rhabdoviridae. In the present invention, preferred paramyxoviruses include viruses belonging to Paramyxovirinae (including Respirovirus, Rubulavirus, and Morbillivirus), more preferably those belonging to the genus Respirovirus (also referred to as Paramyxovirus) or derivatives thereof. The derivatives include viruses that are genetically-modified or chemically-modified in a manner not to impair their gene-transferring ability. Examples of viruses of the genus Respirovirus applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). In the context of the present invention, a more preferred Paramyxovirus is the Sendai virus. These viruses may be derived from natural strains, wild strains, mutant strains, laboratory-passaged strains, artificially constructed strains, or the like.

For example, genes of Paramyxovirinae viruses are commonly listed as follows. In general, NP gene is also listed as "N gene." HN that does not have a neuraminidase activity is listed as "H".

| | | | | | | | |
|---|---|---|---|---|---|---|---|
| Respirovirus | NP | P/C/V | M | F | HN | - | L |
| Rubulavirus | NP | P/V | M | F | HN | (SH) | L |
| Morbillivirus | NP | P/C/V | M | F | H | - | L |

The minus-strand RNA viral vectors can encode in their genomic RNA a desired foreign polypeptide to be used as an antigen. The foreign polypeptide is not particularly limited, so long as it is not possessed by natural minus-strand RNA viruses, and may also be a full-length natural protein, a partial fragment thereof (7 amino acids or more, and more preferably 8, 10, or 15 amino acids or more), a fusion polypeptide thereof with another polypeptide, or the like. A recombinant viral vector encoding a foreign polypeptide can be obtained by inserting in the antisense orientation a gene which encodes the polypeptide into the genome of a minus-strand RNA viral vector. Regarding suitable gene insertion sites, any desired position can be selected in non-protein-coding regions of the viral genome. For example, the gene can be inserted between the 3'-leader region and the viral protein ORF closest to the 3'-end; between the viral protein ORFs; and/or between the viral protein ORF closest to the 5'-end and the 5'-trailer region, in the genomic DNA. In genomes deficient in envelope-constituting protein genes, such as the M, F, or HN genes, a foreign gene may also be inserted into those deficient regions. When introducing a foreign gene into a paramyxovirus, it is preferable to insert the gene such that the length of the polynucleotide fragment to be inserted into the genome is a multiple of six (Kolakofski, D. et al., J. Virol. 1998: 72; 891-899; Calain, P. and Roux, L. J. Virol. 1993: 67; 4822-4830). An E-I-S sequence should be arranged between the inserted foreign gene and the viral ORF. Two or more foreign genes can also be inserted in tandem with intervening E-I-S sequences.

Expression levels of a foreign gene can be controlled using the type of transcriptional initiation sequence added upstream (to the 3'-side of the minus strand (negative strand)) of the gene (WO01/18223). The expression levels can also be controlled by the position at which the foreign gene is inserted in the genome: the nearer the insertion position is to the 3'-end of the minus strand, the higher the expression level; conversely, the nearer the insertion position is to the 5'-end, the lower the expression level. Since a high expression level of antigen polypeptide may be generally advantageous, it is preferable to link the foreign peptide-encoding gene to a highly efficient transcriptional initiation sequence, and to insert it near the 3'-end of the minus strand genome. Specifically, a foreign gene may be inserted between the 3'-leader region and the viral protein ORF closest to the 3'-end. Alternatively, a foreign gene may be inserted between the ORFs of the viral protein gene closest to the 3'-end and the second closest viral protein gene, or between the ORFs of the second and third closest viral protein genes. ' In wild type paramyxoviruses, the viral protein gene closest to the 3'-end of the genome is the N gene, the second closest gene is the P gene, and the third closest gene is M gene. Alternatively, in those cases wherein a high level of expression of the antigen polypeptide is undesirable, the level of viral vector gene expression can be suppressed to obtain an appropriate effect, for example, by inserting the foreign gene at a site as close as possible to the 5'-side of the minus strand genome, or by selecting an inefficient transcriptional initiation sequence.

For example, a desired S sequence of a minus-strand RNA virus may be used as the S sequence to be attached when inserting a foreign gene-encoding nucleic acid into the genome. The consensus sequence 3'-UCCCWWUWC-5' (W=A or C; V= A, C, or G)(SEQ ID NO:1) can be preferably used for Sendai viruses. Particularly preferred sequences are 3'-UCCCAGUUUC-5' (SEQ ID NO: 2), 3'-UCCCACUUAC-5' (SEQ ID NO: 3), and 3'-UCCCACUUUC-5' (SEQ ID NO: 4). When shown as plus strand-encoding DNA sequences, these sequences are 5'-AGGGTCAAAG-3' (SEQ ID NO: 5), 5'-AGGGTGAATG-3' (SEQ ID NO: 6), and 5'-AGGGTGAAAG-3' (SEQ ID NO: 7). A preferred E sequence of a Sendai viral vector is, for example, 3'-AUUCUUUUU-5' (SEQ ID NO: 8) or 5'-TAAGAAAAA-3' (SEQ ID NO: 9) for the plus strand-encoding DNA. An I sequence may be, for example, any three nucleotides, specifically 3'-GAA-5' (5'-CTT-3' in the plus strand DNA).

Recombinant RNA viral vectors may be reconstituted using known methods. For example, such vectors can be produced by the steps of (a) transcribing DNA which encodes the genomic RNA of a minus-strand RNA virus expressing a desired exogenous antigen polypeptide, or the complementary strand thereof (antigenomic RNA, plus-strand), in mammalian cells in the presence of viral proteins constituting RNP containing the genomic RNA of the minus-strand RNA virus, and (b) collecting the produced minus-strand RNA viruses or RNP containing the genomic RNA. The "viral proteins constituting RNP" mentioned above refers to proteins that form RNP together with the viral genomic RNA and constitute a nucleocapsid. These are a group of proteins necessary for genome replication and gene expression, and are typically N (nucleocapsid (also referred to as nucleoprotein (NP))-, P (phospho)-, and L (large)-proteins. Although these notations vary depending on viral species, corresponding proteins are known to those skilled in the art (Anjeanette Robert et al., Virology 247:1-6 (1998)). For example, "N" may be denoted as "NP".

When reconstituting viruses, a minus-strand RNA genome (*i.e*. antisense strand, which is the same as the viral genome) or the plus-strand RNA (antigenome, the complementary strand of the genomic RNA) may be generated as described above. However, in order to increase the efficiency of vector reconstitution, the plus-strand is preferably generated. The viral genomic RNA may be deficient in genes encoding envelope-constituting proteins, so long as it encodes viral proteins required for RNP reconstitution. For example, the genomic RNA does not need to encode viral proteins, such as F, HN, and M, so long as it encodes the N, P, and L proteins. Such defective viruses can amplify the genomic RNA in cells, but do not release infectious virions, and thus are useful as highly safe gene transfer vectors (WO00/70055, WO00/70070, and WO03/025570; Li, H.-O. et al., J. Virol. 74(14) 6564-6569 (2000)). To produce a viral vector, the above envelope-constituting proteins are expressed separately in virus-producing cells to complement particle formation. In order to express viral proteins and RNA genome in cells, a vector linked with DNA that encodes the proteins or genome downstream of an appropriate promoter is introduced into host cells. The promoter used include, for example, CMV promoters and CAG promoters (Niwa, H. et al. (1991) Gene. 108: 193-199, and Japanese Patent Application Kokai Publication No. (JP-A) H3-168087 (unexamined, published Japanese patent application)).

The RNA terminals preferably reflect the terminals of the 3'-leader sequence and 5'-trailer sequence as accurately as possible, as in the natural viral genome. For example, a self-cleaving ribozyme is added at the 5'-end of the transcript to allow the ribozyme to accurately cleave off the end of the minus-strand RNA viral genome (Inoue, K. et al. J. Virol. Methods 107,2003, 229-236). Alternatively, in order to accurately regulate the 5'-end of the transcript, the recognition sequence of bacteriophage RNA polymerase is used as a transcription initiation site, and the RNA polymerase is expressed within a cell to induce transcription. The bacteriophage RNA polymerase used include, for example, those of E. coli T3 phage and T7 phage, and Salmonella SP6 phage (Krieg, P.A. and Melton, D.A. 1987, Methods Enzymol. 155: 397-15; Milligan, J.F et al., 1987, Nucleic Acids Res. 15: 8783-798; Pokrovskaya, I.D. and Gurevich, V.V., 1994, Anal. Biochem. 220: 420-23). Such bacteriophage RNA polymerases can be supplied using, for example, vaccinia viruses expressing the polymerases (Fuerst, T.R. et al., Proc. Natl. Acad. Sci. USA 83, 8122-8126 (1986), or supplied from expression vectors such as plasmids. To regulate the 3'-end of the transcript, for example, a self-cleaving ribozyme is encoded at the 3'-end of the transcript; allowing accurate cleavage of the 3'-end with this ribozyme (Hasan, M. K. et al., J. Gen. Virol. 1997: 78: 2813-2820; Kato, A. et al., EMBO J. 1997, 16: 578-587; and Yu, D. et al., Genes Cells 1997, 2: 457-466). An auto-cleaving ribozyme derived from the antigenomic strand of delta hepatitis virus can be used.

In the reconstitution of viruses in which the envelope-constituting protein genes have been deleted, the infectivity of viruses may be complemented by the deleted envelope-constituting proteins. However, for example, the viruses may also be pseudotyped with envelope proteins other than the deleted proteins. Such an envelope protein used may be, for example, the G protein of vesicular stomatitis virus (VSV) (VSV-G) (J. Virology 39: 519-528 (1981)) (Hirata, T. et al., 2002, J. Virol. Methods, 104:125-133; Inoue, M. et al., 2003, J. Virol. 77:6419-6429; Inoue M. et al., J Gene Med. 2004;6:1069-1081). Genes to be deleted from the genome include, for example, genes of spike proteins such as F, HN, H, and G, genes of envelope-lining proteins such as M, and any combinations thereof. Deletion of a spike protein gene is effective in rendering minus-strand RNA viral vectors nontransmissible, whereas deletion of the gene of an envelope-lining protein such as M protein is effective in disabling the particle formation from infected cells. For example, F gene-defective minus-strand RNA viral vectors (Li, H.-O. et al., J.Virol. 74, 6564-6569 (2000)), M gene-defective minus-strand RNA viral vectors (Inoue, M. et al., J.Virol. 77, 6419-6429 (2003)), and the like are preferably used. For example, the use of an F gene-defective vector provided suppressed pathogenicity in a host mouse, allowing the favorable production of antibodies (see Examples). Moreover, greater safety would be assured with vectors defective in any combination of at least two of F, HN (or H) and M genes. For example, vectors lacking both M and F genes are nontransmissible and defective in particle formation while retaining high level infectivity and gene expression ability.

In an example of the production of F gene-defective recombinant viruses, for example, a plasmid expressing a minus-strand RNA viral genome defective in F gene or a complementary strand thereof is transfected into host cells along with an expression vector expressing F protein and expression vectors for N, P, and L proteins. Alternatively, viruses can be more efficiently produced by using host cells in which the F gene has been incorporated into their chromosomes (WO00/70070). In this case, a sequence-specific recombinase such as Cre/loxP and FLP/FRT and a target sequence thereof are preferably used so that the F gene can be inducibly expressed (see WO00/70055, WO00/70070; Hasan, M. K. et al., 1997, J. General Virology 78: 2813-2820). Specifically, for example, the envelope protein genes are integrated into a vector having a recombinase target sequence, such as the Cre/loxP inducible expression plasmid pCALNdlw (Arai, T. et al., J. Virology 72, 1998, p1115-1121). The expression is induced by, for example, infection with the adenovirus AxCANCre at an MOI of 3 to 5 (Saito et al., Nucl. Acids Res. 23: 3816-3821 (1995); and Arai, T, et al., J. Virol 72, 1115-1121 (1998)).

The minus-strand RNA viruses used in the present invention may be deficient in accessory genes. For example, by knocking out the V gene, one of the accessory genes of Sendai virus (SeV), the pathogenicity of SeV toward hosts such as mice is remarkably reduced without hindering gene expression and replication in cultured cells (Kato, A. et al., 1997, J. Virol. 71:7266-7272; Kato, A. et al., 1997, EMBO J. 16:578-587; Curran, J. et al.; WO01/04272; and EP1067179).

In addition, minus-strand RNA viruses used may include mutations in the P gene or L gene so as to enhance the persistence of infection. Specific examples of such mutations include mutation of Glu at position 86 (E86) of the SeV P protein, substitution of Leu at position 511 (L511) of the SeV P protein to another amino acid, or substitution of homologous sites in the P protein of a different minus-strand RNA virus. Specific examples include substitution of the amino acid at position 86 to Lys, and substitution of the amino acid at position 511 to Phe. Regarding the L protein, examples include substitution of Asn at position 1197 (N 1197) and/or Lys at position 1795 (K1795) in the SeV L protein to other amino acids, or substitution of homologous sites in the L protein of another minus-strand RNA virus, and specific examples include substitution of the amino acid at position 1197 to Ser, and substitution of the amino acid at 1795 to Glu. Mutations of the P gene and L gene can significantly increase the effects of persistent infectivity, suppression of the release of secondary virions, and suppression of cytotoxicity.

The minus-strand RNA virus can further encode a Th2 cytokine and/or an anti-inflammatory cytokine as well as an antigen (Example 11). In the present invention, the term "Th2 cytokine" refers to cytokines that are more predominantly produced in type II helper T cells (Th2 cells) than type I helper T cells (Th1 cells). Specific examples of the Th2 cytokine include IL-4, IL-5, IL-6, IL-9, IL-10, IL-13, and TGF-β. The anti-inflammatory cytokine (also referred to as anti-inflammation cytokine) collectively refers to polypeptides that act to suppress inflammation, and includes signaling molecules that promote signal transduction suppressing inflammation and/or signaling molecules that inhibit signal transduction promoting inflammation (for example, inflammatory cytokine inhibitors). In the present invention, the anti-inflammatory cytokine specifically includes interleukin (IL)-4, IL-10, IL-11, IL-13, TGF-β, soluble TNF-α receptors, and IL-1 receptor antagonists (IL-1ra). The Th2 cytokine and/or anti-inflammatory cytokine to be encoded by vectors may be a full-length natural polypeptide or a partial peptide thereof (such as an active fragment) as long as the activity is maintained. For example, the deletion of amino acid residues (for example, 1 to 30 amino acids, and more specifically 1, 2, 3, 4, 5, 10, 15, 20, or 25 amino acids) at the N terminal and/or C terminal is highly likely not to affect the cytokine activity. Moreover, polypeptides that inhibit the signal transduction of inflammatory cytokines, such as soluble fragments (including a ligand-binding domain) of inflammatory cytokine receptors, antibodies or antibody fragments that bind to the ligand-binding domain of inflammatory cytokine receptors, or the like, may be used. Moreover, it is possible to use desired fragments of Th2 cytokines and/or anti-inflammatory cytokines that include mature polypeptides from which signal sequences have been removed. For N-terminal signal sequence for extracellular secretion, the signal sequence of a desired protein may appropriately be used. The secretion signal sequence that can be used includes, but is not limited to, for example, signal sequences of desired secretory proteins such as interleukin (IL)-2 and tissue plasminogen activator (tPA). Moreover, the Th2 cytokine and/or anti-inflammatory cytokine may be expressed as a fusion protein with other peptides.

The Th2 cytokine in the present invention is more preferably a cytokine selected from the group consisting of IL-4, IL-10, IL-13, and TGF-β, and most preferably IL-10. The nucleotide and amino acid sequences of each cytokine gene are known (IL-4: NM_000589, NP_000580, AAH66277, AAH67515, NP_758858, NP_067258, NP_958427; IL-10: NM_000572, NP_000563, CAG46825, NP_034678, NP_036986; IL-13: NM_002188, NP_002179, AAB01681, NP_032381, NP_446280; TGF-β (transforming growth factor-β): M_60316).

The activity of each Th2 cytokine or anti-inflammatory cytokine can be detected by known methods. Known examples include methods for detecting the activity by proliferation assays using the mouse mast cell MC/9 (ATCC CRL-8306), the human erythroleukemia cell strain TF-1 (ATCC CRL-2003), and the like (Thompson-Snipes, L. et al., 1991, J. Exp. Med. 173:507-510; Kruse N et al., EMBO J. 1993; 12:5121-5129; Oshima Y et al., J Biol Chem 2001;276:15185-91; Oshima, Y., et al., J. Biol. Chem. 275, 14375-14380, 2000; and Leland, P. et al., Oncol. Res. 7,227-235,1995). For example, deletion mutants of various Th2 cytokines or anti-inflammatory cytokines produced by gene recombination techniques can be assayed by this method to identify active fragments.. It is preferably to calculate 50% effective dose (ED₅₀) and use partial peptides whose activity calculated based on the above (for example, the inverse of ED₅₀) is 50% or more as compared with that of the wild type, preferably 60% or more, 70% or more, 80% or more, 90% or more, or 95% or more.

The origin of the Th2 cytokine and/or anti-inflammatory cytokine to be encoded by the vectors is not specifically limited, and accordingly any mammal, such as mouse, rat, guinea pig, rabbit, pig, cow, horse, donkey, goat, dog, chimpanzee, monkey, and human, may be used. However, it is suitable to use the cytokine derived from the same species as the subject to be administered. The nucleotide sequences of nucleic acids encoding these cytokines are available from the databases described above. Specific examples include Genbank Accession Nos. AY410237 and NM_010548 for mouse IL-10, and Genbank Accession Nos.AY029171 and NM_000572 for human IL-10.

In addition, the minus-strand RNA viruses of the present invention may encode a protein associated with a desired disease, or a polypeptide including a partial peptide (for example, partial peptide of 9, 8, 7, 6, or 5 or more amino acids) thereof. Examples of such diseases include neurodegenerative diseases, and proteins associated with the neurodegenerative diseases include, for example, amyloid β.

Regarding more specific methods for the reconstitution of recombinant viruses, one can refer to, for example, the following references: WO97/16539; WO97/16538; WO00/70055; WO00/70070; WO01/18223; WO03/025570; WO2005/071092; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404; Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; Tokusumi, T. et al. Virus Res. 2002: 86; 33-38; Li, H.-O. et al., J. Virol. 2000: 74; 6564-6569. Following these methods, minus-strand RNA viruses including parainfluenza virus, vesicular stomatitis virus, rabies virus, measles virus, rinderpest virus, Sendai virus, and the like can be reconstituted from DNA.

Desired mammalian cells and the like can be used for virus production. Specific examples of such cells include cultured cells, such as LLC-MK2 cells (ATCC CCL-7) and CV-1 cells (for example, ATCC CCL-70) derived from monkey kidney, BHK cells (for example, ATCC CCL-10) derived from hamster kidney, and cells derived from humans. In addition, to obtain a large quantity of a virus vector, a viral vector obtained from an above-described host can be used to infect embryonated hen eggs to amplify the vector. Methods for manufacturing viral vectors using hen eggs have already been developed (Nakanishi, et al., ed. (1993), "State-of-the-Art Technology Protocol in Neuroscience Research III, Molecular Neuron Physiology", Koseisha, Osaka, pp. 153-172). For example, a fertilized egg is placed in an incubator, and cultured for nine to twelve days at 37 to 38°C to grow an embryo. After the viral vector is inoculated into the allantoic cavity, the egg is then cultured for several days (for example, three days) to proliferate the viral vector. Conditions, such as the period of culture, may vary depending upon the recombinant Sendai virus being used. Then, allantoic fluids, including the vector, are recovered. Separation and purification of a Sendai virus vector from allantoic fluids can be performed according to conventional methods (Tashiro, M., "Virus Experiment Protocol," Nagai, Ishihama, ed., Medical View Co., Ltd., pp. 68-73, (1995)).

The recovered viral vectors can be purified to be substantially pure. Purification can be achieved using known purification/separation methods, including filtration, centrifugation, adsorption, and column purification, or any combinations thereof. The phrase "substantially pure" means that the virus component constitutes a major proportion of a solution of the viral vector. For example, a viral vector composition can be deemed "substantially pure" based on the fact that the proportion of protein contained as the viral vector component as compared to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for the paramyxovirus vector include, for example, methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorption to fucose sulfate-containing polysaccharide and/or degradation products thereof (WO97/32010); however, the invention is not limited thereto.

A minus-strand RNA viral vector, a nucleic acid producing such a viral vector, cells into which such a vector or the nucleic acid producing such a vector has been introduced, or a lysate thereof can be combined as desired with pharmaceutically acceptable carriers or media to yield a composition for antibody production. Herein, the term "composition for antibody production" refers to a composition used exclusively for the purpose of antibody production. The term "pharmaceutically acceptable carriers or media" encompasses desired solutions capable of suspending the viral vectors or cells, examples of which include phosphate buffered saline (PBS), sodium chloride solution, Ringer's solution, and culture solution. The present invention relates to methods for producing antigen vector compositions for antibody production, which include the step of producing compositions that include a minus-strand RNA viral vector carrying a nucleic acid that encodes a foreign polypeptide to be used as an antigen, a nucleic acid producing such a viral vector, cells into which such a vector or a nucleic acid producing such a vector has been introduced, or a lysate of the cells; and pharmaceutically acceptable carriers or media. Moreover, the present invention also relates to uses of a minus-strand RNA viral vector, a nucleic acid producing such a viral vector, cells into which such a vector or a nucleic acid producing such a vector has been introduced, or a lysate thereof, in the production of compositions for antibody production. Regarding the production of recombinant minus-strand RNA viral vectors carrying a nucleic acid which encodes a foreign polypeptide to be used as an antigen, one may refer to the above description of the present specification. Immunostimulants, such as cytokines, cholera toxin, and Salmonella toxin, can be added to compositions for antibody production to improve their immunogenicity. Furthermore, the compositions may be combined with adjuvants, such as aluminum potassium sulfate, complete Freund's adjuvant, incomplete Freund's adjuvant, MF59 (oil emulsion), MTP-PE (muramyl tripeptide derived from the cell wall of mycobacteria), QS-21 (derived from soapbark tree *Quilaja* s*aponaria*), and alum (particles of aluminum compounds such as aluminum hydroxide, aluminum sulfate, and aluminum phosphate).

Moreover, the above composition preferably further contains a Th2 cytokine or a nucleic acid encoding a Th2 cytokine. Alternatively, it may contain another anti-inflammatory cytokine or a nucleic acid encoding another anti-inflammatory cytokine, alone or in combination with the above Th2 cytokine or nucleic acid encoding the Th2 cytokine. For the Th2 cytokine and anti-inflammatory cytokine, those described herein or a combination thereof can be used. Preferably, the Th2 cytokine is selected from the group consisting of IL-4, IL-10, and IL-13. Moreover, the above composition may-contain a Th2 adjuvant. For example, the use of a composition containing a Th2 adjuvant can further promote a shift in the Th1/Th2 balance toward Th2. The term "Th2 adjuvant" refers to adjuvants which activate type II helper T cells (Th2 cells) more predominantly than type I helper T cells (Th1 cells). Specifically, aluminum hydroxide (alum), cholera toxin (B subunit), *Schistosoma mansoni* egg extract proteins (such as Lacto-N-fucopentaose III), and the like may be used (Grun, J. L. and P. H. Maurer, 1989, Cellular Immunology 121: 134-145; Holmgren J et al., 1993, Vaccine 11:1179-1184; Wilson AD et al., 1993, Vaccine 11:113-118; Lindsay DS et al, 1994, Int Arch Allergy Immunol 105:281-288; Xu-Amano J et al., 1993, J Exp Med 178:1309-1320; Okano M et al., 2001, J Immunol. 167(1):442-50).

Moreover, organic substances, such as biopolymers, inorganic substances, such as hydroxyapatite, specifically a collagen matrix, polylactic polymer or copolymer, polyethylene glycol polymer or copolymer, chemical derivatives thereof, and the like may also be combined as a carrier.

Antibody production can be induced through the expression of an antigen polypeptide by administering a produced minus-strand RNA virus or a composition containing the vector to an animal. The vector may be administered either in vivo or *ex vivo* via cells. The minus-strand RNA viral vector to be inoculated need not necessarily be an infectious virion, and may be a noninfectious virion or a virus core (RNP complex containing a genome and genome-binding viral proteins) or the like, so long as it maintains an ability to express the carried antigen polypeptide gene from the genomic RNA. In the present invention, the term "minus-strand RNA viral vector" refers to complexes that include a ribonucleoprotein (RNP) complex having the genomic RNA derived from the minus-strand RNA virus and viral proteins necessary for replicating the RNA and expressing the carried gene, and that replicate the genomic RNA and express the carried gene in infected cells. The RNP is, for example, a complex having the genomic RNA of a minus-strand RNA virus or the complementary strand thereof (antigenomic RNA), and N, L, and P proteins. Thus; in the present invention, the minus-strand RNA viral vector includes viral infectious particles, noninfectious particles (virus-like particles; also referred to as VLP), and RNPs containing a genomic RNA and viral proteins binding to the genomic RNA, such as a nucleocapsid of the minus-strand RNA virus. RNP (viral core) that is a virion from which its envelope has been removed is, when introduced into cells, still capable of replicating the viral genomic RNA in the cells (WO97/16538; WO00/70055). RNP or VLP may be inoculated into an animal together with, for example, a transfection reagent (WO00/70055; WO00/76070).

To inoculate via cells, the minus-strand RNA viral vector is introduced into appropriate cultured cells, cells collected from an inoculation subject animal, or the like. For infecting cells with the minus-strand RNA viruses outside the body (for example, in a test tube or dish), the infection is carried out *in vitro* (or *ex vivo),* in a desired physiological aqueous solution such as a culture solution or a physiological salt solution. Herein, MOI (multiplicity of infection; number of infectious viruses per cell) is preferably within a range of 1 to 1000, more preferably 2 to 500, yet more preferably 3 to 300, and even more preferably 5 to 100. The minus-strand RNA viruses and cells can be sufficiently contacted even for a short time, The contact may be carried out, for example, for 1 minute or more, and preferably 3 minutes or more, 5 minutes or more, 10 minutes or more, or 20 minutes or more. The duration may be for example about 1 to 60 minutes, and more specifically about 5 to 30 minutes. Of course, the contact may be carried out for the duration more than the above, such as several days or more.

Known transfection methods can be used to introduce RNPs or non-infectious viral particles (virus-like particles (VLPs)) that contain viral genomic RNA into cells. Specifically, such transfection of cells can be achieved by various techniques known to those skilled in the art, such as methods that utilize calcium phosphate (Chen, C. & Okayama, H. (1988) BioTechniques 6:632-638; Chen, C. and Okayama, H., 1987, Mol. Cell. Biol. 7: 2745), DEAE-dextran (Rosenthal, N. (1987) Methods Enzymol. 152:704-709), various liposome-based transfection reagents (Sambrook, J. et al. (1989) Molecular Cloning: A Laboratory Manual (Cold Spring Harbor Laboratory, Cold Spring Harbor, NY)), or electroporation (Ausubel, F. et al. (1994) In Current Protocols in Molecular Biology (John Wiley and Sons, NY), Vol. 1, Ch. 5 and 9). Chloroquine may be added to the transfection to suppress the degradation in endosomes (Calos, M, P., 1983, Proc. Natl. Acad. Sci. USA 80: 3015). Transfection reagents include, for example, DOTMA (Roche), Superfect Transfection Reagent (QIAGEN, Cat No. 301305), DOTAP, DOPE, DOSPER (Roche #1811169), TransIT-LT1 (Mirus, Product No. MIR 2300), CalPhosTM Mammalian Transfection Kit (Clontech #K2051-1), and CLONfectinTM (Clontech #8020-1). Enveloped viruses are known to incorporate host cell-derived proteins during virion formation, and such proteins can potentially cause antigenicity and cytotoxicity when introduced into cells (J. Biol. Chem. (1997) 272,16578-16584). It is thus advantageous to use RNPs without the envelope (WO 00/70055).

Moreover, virus RNPs can be directly produced in a cell by introducing into the cell an expression vector which expresses viral genomic RNAs and an expression vector which encodes viral proteins (N, P, and L proteins) necessary for replicating the genomic RNAs. Cells into which viral vectors have been introduced may also be produced in such a manner.

After cells into which minus-strand RNA viral vectors have been introduced have been prepared, the cells are cultured for about 12 hours to 5 days (preferably 1 to 3 days) to express antigen polypeptides. The obtained cells are then inoculated into an animal, either directly or in the form of a lysate prepared by lysis. The lysate of the vector-infected cells can be produced by a method in which the cell membranes are lysed with a surfactant, a method in which freezing and thawing are repeated, or the like. As the surfactant, nonionic Triton X-100, Nonidet P-40, or the like are used at a concentration of 0.1 to 1%. For example, such a lysate can be obtained by washing cells with PBS, collecting the cell aggregation by centrifugation, and then resuspending it in TNE buffer [25mM Tris-HCl (pH7.5), 150mM NaCl, 1mM EDTA, and 1% Nonidet P-40], and left on ice for 10 to 30 minutes. If a protein to be used as an antigen is soluble in cytoplasm, a supernatant, prepared by centrifuging the obtained lysate (10,000 x g for 10 minutes) and removing an unnecessary insoluble fraction as a precipitate, can be used for immunization. In situations where use of a surfactant is undesirable, lysate to be administered to sites can be prepared by destroying the cells that were washed and resuspended in PBS by freezing and thawing repeatedly five to six times. Methods for producing antibodies or antibody-producing cells of the present invention may further include, prior to inoculation into animals, the step of preparing minus-strand RNA viral vectors carrying nucleic acids which encode foreign polypeptides to be used as antigens, nucleic acids producing such viral vectors, cells into which such vectors or the nucleic acids producing such vectors have been introduced, or lysates of the cells, and any combination thereof.

Moreover, antibody production may be induced by administering to an animal nucleic acids which produce a minus-strand RNA viral vector carrying nucleic acids that encode a foreign polypeptide to be used as an antigen, and thereby producing a minus-strand RNA viral vector that expresses a target antigen polypeptide in the animal. The term "nucleic acids which produce a minus-strand RNA viral vector" refers to a set of nucleic acids which express RNAs and a group of proteins necessary for producing a recombinant of the minus-strand RNA viral vector. More particularly, this term refers to a set of nucleic acids including nucleic acids encoding genomic RNAs of a minus-strand RNA virus which encode a desired foreign polypeptide, or the complementary strand thereof (antigenomic RNAs; plus-strand), and nucleic acids encoding a group of viral proteins constituting RNPs which include genomic RNAs of a minus-strand RNA virus. These nucleic acids include appropriate promoters so that the encoded RNAs or proteins may be expressed. Specific examples suitable for use include a CMV promoter (Foecking, M.K, and Hofstetter H. Gene 1986; 45: 101-105), a retrovirus LTR (Shinnik, T. M.; Lerner, R. A. & Sutcliffe (1981) Nature, 293, 543-548), an EF1 promoter, a CAG promoter (Niwa, H. et al. (1991) Gene. 108: 193-199; JP-A H3-168087) and the like Plasmid vectors can suitably be used as nucleic acids. Preferably, the genome of the minus-strand RNA virus carries genes that encode a group of viral proteins (typically, N, L, and P) constituting RNPs which include the genomic RNAs, and all envelope-constituting protein genes that the wild type virus of the parent strain contains. If the genome of the minus-strand RNA virus is defective in envelope-constituting protein genes, nucleic acids encoding envelope proteins (for example, defective envelope proteins in the genome, or other envelope proteins such as VSV-G) which complement the infectious virion formation may be included in the set of nucleic acids mentioned above. By administering these nucleic acids to an animal, recombinant minus-strand RNA viruses are produced in the animal. The term "group of viral proteins constituting RNPs which include genomic RNAs of a minus-strand RNA virus" refers to proteins which form RNPs together with viral genomic RNAs to constitute nucleocapsids. These are a group of proteins necessary for replicating the genome and expressing genes, and typical examples are N, P, and L proteins. That is, the present invention also relates to methods including the steps of: (a) inoculating animals with (i) nucleic acids encoding genomic RNAs of minus-strand RNA viruses which encode foreign polypeptides, or the complementary strand thereof and (ii) nucleic acids each encoding N, P, and L proteins; and (b) collecting antibodies or antibody-producing cells from the animals.

In step (a), rather than directly inoculating the nucleic acids into animals, cells into which the nucleic acids producing the viral vectors have been introduced can be previously prepared, and then the cells, lysates of the cells, or antigens produced from the lysates of the cells can be inoculated. For details of nucleic acids producing minus-strand RNA viral vectors, see the following references: WO97/16539; WO97/16538; WO00/70055; WO00/70070; WO01/18223; WO03/025570; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T, 1997, J. Virol. 71: 1265-1271; Bridgen, A. and Elliott, R. M., 1996, Proc. Natl. Acad. Sci. USA 93: 15400-15404; Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; Tokusumi, T. et al. Virus Res. 2002: 86; 33-38, Li, H.-O. et al., J. Virol. 2000: 74; 6564-6569.

The administration route is not particularly limited; however, suitable examples are intramuscular (for example, gastrocnemius) injection, subcutaneous administration, nasal administration, intracutaneous administration in palm or foot pad, direct splenic administration, and intraperitoneal administration. Nasal administration includes the situation wherein an infected area remains within the nasal cavity (intranasal administration), the situation wherein the area extends to the airway or lung (transnasal-pulmonary administration), and so on.

Moreover, oral administration, transanal (transrectal) administration, or intrauterine administration (via urinary organ or reproductive organ) may be adopted, exemplary target organs including the oral cavity, intestinal tract, urogenital organs, and upper airway mucosa. In oral administration, infection of a target organ can be achieved by processing the vector into a sustained-release microcapsule or the like so as to retain the carrying capacity even when exposed to highly acidic gastric juice.

The inoculation site may be at one or more positions (for example, 2 to 15 positions), and the inoculation dose may be appropriately adjusted according to the animal to be inoculated, the inoculation site, the frequency of inoculation and the like. For example, the inoculation dose per site may be a virus titer of 1 x 10⁴ CIU (cell infectious unit) to 5 x 10¹¹ CIU, and preferably 1 x 10⁶ CIU to 1 x 10¹⁰ CIU. For inoculation via cells (*ex vivo* administration), a cultured cell strain (such as a sarcoma cell strain) from the same species can be infected with a minus-strand RNA viral vector to inoculate 10⁴ to 10⁹ cells, preferably 10⁵ to 10⁸ cells, or a lysate thereof into an animal. Although only the priming can induce a significant antibody titer, booster with twice or more inoculations can further increase an antibody titer.

As a minus-strand RNA viral vector encoding an antigen, those further encoding a Th2 cytokine and/or an anti-inflammatory cytokine can be suitably used. Alternatively, when immunizing with a minus-strand RNA viral vector encoding an antigen, and/or before or after that (for example, within 1 to 72 hours, preferably 1 to 48 hours, and preferably 1 to 24 hours, before or after the administration of the minus-strand RNA viral vector encoding an antigen), a Th2 cytokine and/or an anti-inflammatory cytokine, or a vector encoding them may be administered. The administration site is not particularly limited; however, the administration preferably involves delivery to the same place as that for a minus-strand RNA viral vector encoding an antigen, or in the near vicinity (within 1 mm to 3 cm, and preferably within 3 mm to 10 mm).

When inoculating an animal with nucleic acids producing a minus-strand RNA viral vector, the nucleic acids encoding the genomic RNAs of the minus-strand RNA virus which encode foreign polypeptides, or the complementary strand thereof, and nucleic acids encoding N, P, and L proteins can each preferably administered at a weight ratio of 5: 0.5: 0.5: 2 into the animal; however, the weight ratio of the respective nucleic acids may be appropriately adjusted. For example, when an expression plasmid is used, 5 µg to 1000 µg of a plasmid encoding the viral genomic RNAs (plus-strand or minus-strand), 0.5 µg to 200 µg of an N expression plasmid, 0.5 µg to 200 µg of a P expression plasmid, and 2 µg to 500 µg of an L expression plasmid can be administered. The nucleic acids are administered by injecting naked DNAs, or by injecting a mixture with a transfection reagent, for example. Examples of a transfection reagent include lipofectamine and polycationic liposome, and specifically DOTMA (Roche), Superfect (QIAGEN #301305), DOTAP, DOPE, DOSPER (Roche #1811169), TransIT-LT1 (Mirus, Product No. MIR 2300), or the like can be used.

Booster dose can be delivered by inoculation methods similar to the above, in one to several weeks (for example, after one to three weeks, and more specifically about two weeks) from the priming. Suitable examples include booster by intraperitoneal administration and/or intracutaneous injection to the dorsum, foot pad, or the like. To further increase an antibody titer, booster doses may be subsequently repeated. In booster doses, the above-mentioned minus-strand RNA viral vector carrying nucleic acids which encode a foreign polypeptide to be used as an antigen, nucleic acids producing such viral vectors, cells into which such vectors or the nucleic acids producing such vectors have been introduced, a lysate of the cells, or an antigen purified from the lysate of the cells are preferably inoculated into an animal. The cell lysate for booster immunization can be prepared as mentioned above. The purification degree of antigen is not particularly limited, and may vary. For example, an antigen purified from a lysate by centrifugation, filtration, dialysis, various types of chromatography or the like, can be used. Moreover, it is suitable to inoculate with a foreign polypeptide to be used as an antigen or a partial peptide thereof. For example, a partial peptide can be synthesized and appropriately conjugated with a carrier protein such as keyhole limpet hemocyanin (KLH), bovine serum albumin (BSA), thyroglobulin (THY), and ovalbumin (OVA), to use in immunization. The length of the partial peptide is preferably, for example, 8 to 25 residues (9 to 20, 10 to 18, or 12 to 16 residues). A Th2 cytokine and/or an anti-inflammatory cytokine or a vector encoding them may also be administered with a booster.

After a sufficient antibody titer is obtained, antibodies are collected by collecting blood (in the case of polyclonal antibodies). When preparing monoclonal antibodies, cells are collected from the spleen, lymph node or such of an immunized animal, and immortalized by procedures such as cell fusion with a myeloma to produce hybridomas, and then cells producing the target antibodies are selected and cloned (Harlow, E., 1988, Antibodies; A Laboratory Manual, Cold Spring Harbor, New York; John E. Coligan et al. (Eds), Current Protocols in Immunology (Chapter 2); Peter J. Delves (Ed), Antibody Production: Essential Techniques, 1997, John Wiley & Sons; R. Kontermann & S. Dubel (Ed), Antibody Engineering, 2001, Springer Verlag, Heidelberg; Caponi, L. & Migliorini, P. (Eds), Antibody Usage In The Lab, Springer Lab Manual, 1999, Springer-Verlag Berlin and Heidelberg GmbH & Co.; William C. Davis (Ed.), Monoclonal Antibody Protocols., Methods in Molecular Biology, Vol. 45, 1995, Humana Press Inc., Totowa, N.J.). Examples of animals to be inoculated include desired nonhuman vertebrates that express antibodies, such as fishes, amphibians, reptiles, birds, and mammals, preferably birds and nonhuman mammals, and more preferably nonhuman mammals. Specific examples include a chicken, rabbit, goat, sheep, pig, cow, and rodents such as a mouse and rat, and nonhuman primates such as a monkey, and other mammals.

The antibody titer can be measured by an ELISA (Enzyme-linked immunosorbent assay) or using the Ouchterlony method. In one example of ELISAs, after an antigen is adsorbed onto a microplate, a produced antiserum is subjected to 1000- to 2-fold serial dilution, and then added to the plate to perform an antigen-antibody reaction. Furthermore, a peroxidase-labeled antibody from a heterologous animal against an antibody of the immunized animal is reacted as a secondary antibody, and then subjected to color development. The antibody titer can be calculated as the dilution ratio exhibiting half maximum absorbance. Moreover, in the Ouchterlony method, using a phenomenon in which an antigen and antibody are diffused in an agar gel to exhibit an immunoprecipitation reaction and generate a white precipitation line, the dilution ratio of the antibody at which the immunoprecipitation reaction is exhibited can be measured as the antibody titer.

Antibodies may be prepared in the form of a serum or diluted solution, or may be purified. For example, a desired class such as IgG and IgM antibodies can be purified. For example, to purify IgG antibodies, which are most frequently used because of their high affinity in general, a column or beads in which protein A or protein G has been immobilized on a carrier can be used. IgM antibodies can be purified using a column containing 2-mercaptopyridine as a ligand. The 2-mercaptopyridine column can also be used for purifying IgY (chicken egg yolk antibodies) (HiTrap IgY Purification HP; Amersham Biosciences K.K.). Alternatively, antibodies can be purified by DEAE anion exchange chromatography. For example, when a large amount of antibody is purified from a goat, sheep or the like, salting-out is first performed with a sulfate to yield a crude IgG fraction, and the fraction is passed through a DEAE-cellulose column and then collected as the flow-through fraction without being adsorbed to the column. Moreover, as a method for specifically purifying antibodies from an antiserum, an affinity purification method may also be used. In this method, an antigen is immobilized on a carrier gel to produce an affinity gel, the antiserum is then passed through the gel, and antibodies binding to the antigen can be specifically collected by recovering IgGs that bind to the antigen. The purity of the collected antibodies can be analyzed with HPLC (for example, TSK G3000 SWXL column manufactured by Tosoh Corporation) or the like (Howard, C.C. and Bathell, D.R. eds., Basic Methods in Antibody Production and Characterization, 2001, CRC Press BocaRaton, London, New York).

For selecting or purifying antibodies or antibody-producing hybridomas produced by the methods of the present invention, antibodies which do not bind to a target antigen polypeptide can be removed by negative selection. For example, antibodies that bind to minus-strand RNA virus-derived viral proteins can be removed from the produced antibody pool. In this case, for example, a minus-strand RNA viral vector which does not encode a target antigen polypeptide, cells into which such a viral vector has been introduced, a lysate of the cells, or one or more of viral proteins from the virus can be prepared to remove antibodies binding thereto. Specifically, a solution containing antibodies is mixed with a carrier gel on which the minus-strand RNA viral vector which does not encode a target antigen polypeptide, a cell into which such a viral vector has been introduced, a lysate of the cell, or a viral protein from the virus has been immobilized, and then antibodies binding to it are removed. Since antibodies binding to the minus-strand RNA viral vector are removed using the above procedure, the purity of the antibodies binding to the target antigen polypeptide can be increased by collecting the remaining solution. Similar procedures can be applied to hybridomas or the like. For example, a solution containing antibodies produced by hybridomas is mixed with a minus-strand RNA viral vector which does not encode a target antigen polypeptide, a cell into which the viral vector has been introduced, a lysate of the cell, or a viral protein from the virus, and then the binding is detected. By selecting hybridomas producing antibodies which do not bind to them, hybridomas producing antibodies that bind to the viral vector itself can be excluded.

The obtained antibodies can be preserved in the form of a solution or freeze-dried product. In the case of a solution, sodium azide may be added as a preservative. Moreover, bovine serum albumin (BSA) may be contained as a stabilizer. For example, antibodies can be diluted with phosphate buffered saline containing 2 mg/ml of bovine serum albumin and 0.1 % of sodium azide. Examples of uses of antibodies include analysis of a surface antigen by flow cytometry, analysis of an intracellular antigen by flow cytometry, immunohistochemical staining, immunoblotting (Western blotting), immunoprecipitation, ELISA, ELISPOT, *in vivo* assay in a mouse or another mammal, bioassay, blocking, neutralization assay, and antibody treatment" but are not limited thereto. For use in bioassay or *in vivo,* antibodies that do not include a preservative such as sodium azide are preferred. When used for protein detection, antibodies can be appropriately labeled. For example, various labeling methods such as enzyme labeling, fluorescent labeling, radioactive labeling, biotinylation, and colloidal gold labeling are known (P. Cuatrecasas, et al., Biochemistry, 11, 2291 (1972); S. Yoshitake, et al., Eur. J. Biochem., 101, 395 (1979); M. J. O'Sullivan, et al., Anal. Biochem. 100,100 (1979); S. Yoshitake, et al., Anal. Lett., 15, 147 (1982); J. V. Staros, Biochemistry, 21, 3950 (1982); E. Ishikawa, et al., J. Immunoassay, 4, 209 (1983); S. Yoshitake, et al., Scand. J. Immunol.,10, 81 (1979); K. Fujiwara, et al., J. Immunol. Methods, 112, 77 (1988)). When antibodies are labeled with a fluorochrome, FITC, PE, ECD (PE-TxRED), PC5 (PE-Cy5), PC5.5 (PE-Cy5.5), PC7 (PE-Cy7), APC, APC5.5 (APC-Cy5.5), APC7 (APC-Cy7), Cy5, Alexa Fluor^{™} and the like can be used as a fluorochrome, for example. Antibodies may be fragmented into the form of F(ab')₂, Fab', Fab, Fv or the like. Antibody fragments can be obtained by decomposing antibodies with a peptidase such as papain or pepsin.

### Examples

Hereinafter, the present invention is specifically described with reference to Examples; however, it should not be construed as being limited thereto. All the references cited herein are incorporated as parts of the present specification.

### [Example 1] Production of anti-LacZ antibodies by administration of a minus-strand RNA viral vector carrying the LacZ gene

### 1. Immunization

An F gene-defective Sendai virus vector expressing LacZ, SeV18+LacZ/ΔF (WO00/70070), prepared at 2 x 10⁸ CIU/ml with a physiological saline, was administered to the gastrocnemius muscle of six male BALB/cA mice (six-week-old). Two sites on one side of the muscle were inoculated by intramuscular injection at a dose of 100 µl per site. After five weeks from the vector inoculation, blood was collected from the orbital venous plexus, and plasma was separated.

### 2. Detection of antibodies

For antibody assay, 4 µg per lane was subjected to SDS electrophoresis using a commercially available *Escherichia* coli-derived β galactosidase (Sigma G5635). The electrophoresed proteins were transferred to a PVDF membrane with a semi-dry blotter, and blocked with 5% skim milk. The plasma of immunized mice was diluted 200-fold in PBS containing 4% bovine serum albumin, then used for primary antibody reaction. As a positive control, an anti-LacZ antibody (Promega Z378A) diluted 5000-fold in PBS containing 4% bovine serum albumin was used. As a secondary antibody, an HRP-labeled anti-mouse Igs (Biosource AMI4704) diluted in TBS containing 5% skim milk and 0.1% Tween 20 was used. Signals were detected by chemilumiriescence using an ECL plus (Amersham) and an LAS1000 analyzer (FUJI FILM).

### [Results]

With only a single priming using SeV18+LacZ/ΔF, the production of anti-LacZ antibodies was confirmed in the mouse blood after five weeks (Fig. 1).

### [Example 2] Production of anti-GFP antibodies by administration of a minus-strand RNA viral vector carrying the GFP gene

### 1. Immunization

### (1) Priming

A: An F gene-defective Sendai virus vector expressing GFP, SeV18+GFP/ΔF (WO00/70070), prepared by dilution with a physiological saline, was administered to sixteen female BALB/cA mice (six-week-old) at 5 x 10⁶ CIU per mouse by nasal administration, intramuscular injection, direct splenic injection, or foot pad injection (four mice per administration route). In nasal administration, mice under mild anesthesia with sevoflurane were allowed to nasally inhale 100 µl of the vector suspension gradually by spontaneous breathing. In intramuscular injection, 50 µl of the vector suspension was infected into each of two sites in the gastrocnemius muscle of one leg. In direct splenic injection, 50 µl of the vector suspension was injected as it was. In foot pad injection, a total of 50 µl of the vector suspension was injected into both legs, at a dose of 25 µl per leg.

B: The viral vector SeV18+GFP/ΔF, prepared at 1 x 10⁸ CIU/ml with a physiological saline, was administered to the abdominal cavity or the subcutaneous area in the lumbar region of two female six-week-old BALB/cA mice. 100 µl of the vector suspension was injected into the abdominal cavity as it was, and 50 µl of the suspension was subcutaneously administered to each of two sites in the lumbar region.

### (2) Booster

A: Two weeks after priming, all mice were intraperitoneally administered 100 µl of the viral vector SeV18+GFP/ΔF prepared at 5 x 10⁷ CIU/ml. Unboosted control mice were administered 100 µl of a physiological saline in the same manner.
B: Sixteen days after priming, all mice were intraperitoneally administered 100 µl of the viral vector SeV 18+GFP/ΔF prepared at 1 x 10⁸ CIU/ml.

### (3) Blood collection

A: Prior to the priming (nonimmunized control), and on day 28 after the priming, about 100 µl of blood was collected from the orbital vein using a heparin-coated capillary, and plasma was separated.
B: On day 19 after the priming (after three days from the booster), about 100 µl of blood was collected from the orbital vein similarly to the above, and serum was separated.

### (4) Detection of antibodies

A: The anti-GFP antibody titer in the plasma was detected by Western blotting for a commercially available purified GFP protein which had been transferred and immobilized to a PVDF membrane after SDS-PAGE. As controls, a commercially available anti-GFP antibody and nonimmunized mouse plasma were used.
B: The anti-GFP antibody titer in the plasma was detected by Western blotting for the lysate proteins of LLC-MK₂ cells infected with the Sendai virus vector carrying the GFPgene which had been transferred and immobilized onto a PVDF membrane after SDS-PAGE.

### 2. Preparation of a cell lysate for Western blotting

LLC-MK₂ cells were infected with SeV18+ GFP/ΔF at an MOI of 10 in a 10cm dish. After two days, these cells were washed twice with PBS at 4°C. 5 ml of a cell lysis buffer (PBS containing 10mM CHAPS, 2mM EDTA, and 2mM PMSF) was added and left to stand for 20 minutes at 4°C. Soluble materials were collected with a cell scraper. The collected materials were centrifuged at 9000 rpm. The supernatant was then collected and placed in a dialysis tube, Slide-A-Lyzer 2000MW (Pierce Chemical Company), and dialyzed against PBS at 4°C to remove CHAPS. The soluble proteins were quantified by the Bradford method (Bio-Rad DC Protein Assay kit) applied at 5 µg per lane, and then subjected to SDS-PAGE.

### 3. Western blotting

The proteins were transferred to a PVDF membrane with a semi-dry blotter, and blocked with 5% skim milk or 4% bovine serum albumin. The plasma was diluted 200-fold with PBS containing 4% bovine serum albumin and then used for primary antibody reaction. As a positive control, an anti-GFP antibody (Invitrogen) diluted 5000-fold in PBS containing 4% bovine serum albumin was used. As a secondary antibody, an HRP-labeled anti-mouse Igs (Biosource AMI4704) diluted in TBS containing 5% skim milk or 4% bovine serum albumin and 0.1 % Tween 20 was used. Detection was performed by chemiluminescence using an ECL plus (Amersham) and an LAS 1000 analyzer (FUJI FILM).

### [Results]

With a priming using SeV18+GFP/ΔF, the production of anti-GFP antibodies was confirmed in the mouse blood after four weeks, regardless of booster (Fig. 2). The increase in the antibody titer was particularly remarkable in mice inoculated by nasal administration.

### [Example 3] Production of antibodies by intraperitoneally administering a lysate of cells infected with a minus-strand RNA viral vector

### 1. Immunization

### (1) Priming

Two female six-week-old BALB/cA mice were intraperitoneally administered a lysate of mouse sarcoma cells, Meth-A (RIVEN RCB0464; Old, L. et al. (1962) Ann. N.Y Acad. Sci. 101, 80-106) infected with the Sendai virus vector SeV18+GFP/ΔF, at a dose of 5 x 10⁶ cells per mouse. Meth-A cells were infected with the Sendai virus vector SeV18+GFP/ΔF at an MOI = 1,000. After three days, the cells were collected by centrifugation, washed with DPBS(-), and subjected to freezing and thawing repeatedly five times. The cell debris was then removed by centrifugation and the resulting supernatant was used as a lysate.

### (2) Booster

Two weeks after priming, all mice were intraperitoneally administered 100 µl of the viral vector SeV18+GFP/ΔF prepared at 5 x 10⁷ CIU/ml. The unboosted control mice were administered 100 µl of physiological saline in the same manner.

### 2. Detection of antibodies

### (1) Blood collection

Prior to the priming (nonimmunized control), and on days 14 (prior to booster) and 28 after the priming, about 100 µl of blood was collected from the orbital vein using a heparin-coated capillary, and plasma was then separated.

### (2) Western blotting

The anti-GFP antibody titer in the plasma was detected by Western blotting for a commercially available purified GFP protein (Wako chemicals) which had been transferred and immobilized to a PVDF membrane after SDS-PAGE as described in Example 2. As controls, a commercially available anti-GFP antibody (Invitrogen) and the nonimmunized mouse plasma were used.

### [Results]

With a priming using the SeV18+GFP/ΔF-infected cell lysate, the production of anti-GFP antibodies was confirmed in the mouse blood after four weeks, regardless of booster (Fig. 3).

### [Example 4] Production of antibodies by a combination of priming with a viral vector and booster with the vector-infected cell lysate

### 1. Immunization

### (1) Priming

The Sendai virus vector SeV18+GFP/ΔF prepared by dilution with a physiological saline was administered at a dose of 5 x 10⁶ CIU per mouse to a total of eight female six-week-old BALB/cA mice (two mice per administration route) by nasal administration, intramuscular injection; direct splenic injection, and foot pad injection, in the same manner as described in Example 2.

### (2) Booster

Two weeks after the priming, all mice were intraperitoneally administered a lysate of mouse sarcoma cells Meth-A (RIKEN RCB0464) infected with the Sendai virus victor SeV 18+GFP/ΔF, at 5 x 10⁶ cells per mouse. The lysate was produced in the same manner as described in Example 3.

### 2. Detection of antibodies

### (1) Blood collection

Prior to the priming (nonimmunized control), and on days 14 (prior to booster) and 28 after the priming, about 100 µl of blood was collected from the orbital vein using a heparin-coated capillary, and plasma was separated.

### (2) Western blotting

The anti-GFP antibody titer in the plasma was detected by Western blotting for a commercially available purified GFP protein which had been transferred and immobilized to a PVDF membrane after SDS-PAGE. As controls, a commercially available anti-GFP antibody (Invitrogen) and nonimmunized mouse plasma were used (Fig. 4).

### [Results]

With a priming using the SeV 18+GFP/ΔF vector followed by the booster using the cells infected with the vector, the enhanced production of anti-GFP antibodies was confirmed in the mouse blood (Fig. 4).

### [Example 5] Production of anti-GFP monoclonal antibodies

### 1. Immunization

### (1) Priming

One female six-week-old BALB/cA mouse was intraperitoneally administered 100 µl of a viral vector SeV18+GFP/ΔF prepared at 1 x 10⁸ CIU/ml with a physiological saline.

### (2) Booster

Sixteen days after priming, the mouse was intraperitoneally administered 100 µl of the viral vector SeV 18+GFP/ΔF prepared at 1 x 10⁸ CIU/ml with a physiological saline.

### 2. Production of hybridomas

Myeloma cells (P3-X63-Ag8.653) (RIKEN RCB0146) were cultured in a RPMI1640 medium containing 10% fetal bovine serum (FBS), and prepared so that the day of fusion would be in the logarithmic growth phase. On day 3 after the booster, a mouse was sacrificed by cervical dislocation, and the spleen was excised. Splenocytes were isolated by filtration in a serum-free RPMI1640 medium using a sterilized stainless mesh. The splenocytes were washed three times, and the myeloma cells were washed twice, with the serum-free RPMI1640 medium. The both cells were mixed at a cell number ratio of 1:1, and then centrifuged. The supernatant was removed, and then the splenocytes and myeloma cells were fused using 50% polyethylene glycol PEG (Sigma Hybri-Max mw3000-3700). The fused cells were washed with the serum-free medium, and centrifuged twice. The cells were then suspended in 40 ml of RPMI1640 medium containing 20% FBS, 10 mM HEPES, and 1 mM sodium pyruvate, and seeded in a 96-well plate at about 10⁵ cells per well. On the next day of the fusion, 100 µl of HAT selection medium was added to each well. From the next day, the half amount of the medium supernatant was removed, and 100 µl of HAT medium (RPMI1640 containing 20% FBS/1x HAT supplement Hybri-Max (Sigma H0262)) was added. The half-volume medium change was carried out with the HAT selection medium everyday for one week after the cell fusion, and thereafter with an HT medium (RPMI1640 containing 20% FBS/1x HT supplement Hybri-Max (Sigma H0137)) every second day or according to the concentration of living cells. After the fused cells selected by the HAT selection medium grew to the extent that the cells cover approximately half or more of the bottom surface of each well, the culture supernatant was collected and Western blotting was performed to select the fused cells producing anti-GFP antibodies. The proliferated fused cells were cloned by the limiting dilution method using a 96-well plate in which BALB/cA mouse thymocytes were used as feeder cells. After the obtained clones proliferated to the extent that the cells cover approximately the half of each well, the culture supernatant was collected and clones producing anti-GFP antibodies were collected by Western blotting. As a result, the production of monoclonal antibodies recognizing GFP was succeeded using the mouse splenocytes immunized with the Sendai virus vector carrying GFP (Fig. 5).

### [Example 6] Production of monoclonal antibodies recognizing human immunodeficiency virus type 1 (HTV-1) envelope protein gp160

### 1.Immunization

### (1) Priming

A total of eight female seven-week-old BALB/cA mice were administered the F gene-defective Sendai virus vector SeV18+GP160/ΔF carrying human immunodeficiency virus type 1 (HTV-1) envelope protein gp160 gene that had been prepared with a physiological saline at 5 x 10⁸ CIU/ml, under anesthesia with sevoflurane, at a dose of 100 µl per mouse by nasal administration.

### (2) Booster

On days 14, 28, 42, and 56 after priming, the SeV18+GP1160/ΔF-infected 7T1 cell lysate that had been produced by the freezing and thawing method was intraperitoneally administered at a dose of 100 µl per head (5 x 10⁶ cells/100 µl).

### 2. Production of hybridomas

Myeloma cells (P3-X63-Ag8.653) were prepared in the method described in Example 5. On day 4 after the final booster, four mice that have a high anti-gp160 antibody titer in the plasma collected at the final booster were sacrificed by cervical dislocation, and the spleens were excised. Splenocytes were isolated and collected by grinding the spleens on a nylon mesh in a serum-free RPMI1640 medium. The cell fusion of splenocytes and myeloma cells, and the culture of the fused cells were conducted in the methods described in Example 5.

After the fused cells selected by the HAT selection medium grew to the extent that the cells cover approximately half of the bottom surface of each well, the culture supernatant was collected and an ELISA method was performed using a 96-well plate on which the SeV18+GP160/ΔF vector-infected LLC-MK₂ cell lysate was immobilized and a 96-well plate on which a lysate of LLC-MK₂ cells infected with the empty SeV18+/ΔF vector carrying no gene was immobilized. The antibody-producing cells were identified by detecting culture supernatants showing positive reaction only in the 96-well plate on which the SeV18+GP160/ΔF vector-infected LLC-MK₂ cell lysate was immobilized. Whether or not the antibodies are specific to gp160 was confirmed by Western blotting using a PVDF membrane to which the SeV18+GP160/ΔF vector-infected LLC-MK₂ cell lysate produced by the freezing and thawing method had been transferred after SDS-PAGE, in the same manner as described in Example 2 (Fig. 6). The subclass of anti-gp160 antibodies produced by thus obtained hybridomas was determined as IgG₁, using a commercially available kit (Mouse Immunoglobulin Screening/Isotyping Kit; Genzyme Corporation. Cat. No. 97-6550) (Fig. 7). Antibodies to viral proteins of HIVs or other viruses are useful for examination/diagnosis of virus infection, for example.

### [Example 7] Production of monoclonal antibodies recognizing human immunodeficiency virus type 1 (HTV-1) envelope protein gp160, by booster with peptides

### 1. Immunization

### (1) Priming

Priming was performed in the same manner as described in Example 6.

### (2) Booster

Three types of peptides constituting a part of the amino acid sequence of the gp 160 protein were artificially synthesized, and bound to a carrier protein KLH (keyhole limpet hemocyanin) using a usual method. The amino acid sequences of the synthesized peptides are listed below. The numbers in brackets refer to the amino acid numbers of corresponding parts in the gp160 amino acid sequence registered in Genbank (accession number NP_057856). However, cysteines at the amino terminus of #493 and #495 and a cysteine at the carboxy terminus of #494 were added for binding with KLH, and are not included in the gp160 sequence.
#493 CTRKRIRIQRGPGRAFV (303-318) (SEQ ID NO: 10)
#494 SELYKYKVVKIEPLGVC (481-496) (SEQ ID NO: 11)
#495 CPRGPDRPEGIEEEGGER (724-740) (SEQ ID NO: 12)

An emulsion containing a Freund's incomplete adjuvant and a mixture consisting of equal weight of the above-mentioned three types of peptides (50 µg each per head) was produced immediately before booster, and then intracutaneously injected into the dorsum at a dose of 200 µl per head on days 14,28,42, and 56 after the priming.

### 2. Production, selection, and Western blotting of hybridoma

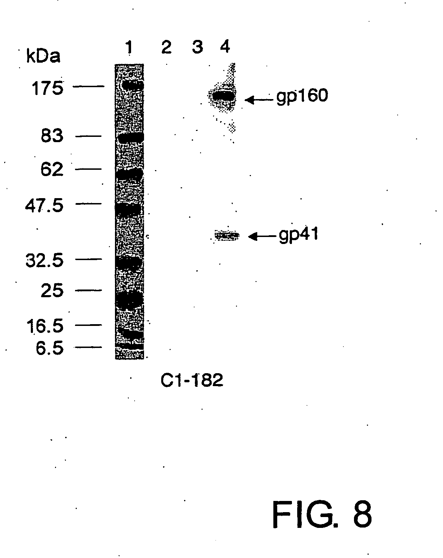

Among the fused cells produced from the mice boosted with peptides in the same manner as described in Example 6, the hybridoma C1-182 producing an anti-gp160 antibody was detected (Fig. 8). The subclass of C1-182 was determined as IgG₁, by a commercially available kit (Mouse Immunoglobulin Screening/Isotyping Kit; Genzyme Corporation. Cat. No. 97-6550) in the same manner as described in Example 6. Accordingly, it was shown that booster effective for the production of a monoclonal antibody can be performed not only with a cell lysate but also with synthetic peptides.

### 3. Epitope confirmation by antibody binding inhibition

Among the peptides used for booster, the peptide which was recognized as an epitope by the antibody produced by the hybridoma C1-182, was determined by binding inhibition assay. Western blotting was performed in the same manner as the above, except that a reaction solution previously mixed with any one peptide of #493, #494, and #495 at 0.1, 1.0, or 10 µg/ml was used for the reaction with the supernatant of hybridoma C1-182. The reaction between the antibody in the supernatant and gp160 on the membrane was inhibited only when the peptide #495 was mixed, showing that the antibody produced by C1-182 recognized the amino acid sequence of the peptide #495 as an epitope. Therefore, it was supported that the booster with peptides was effective (Fig. 9).

### [Example 8] Cloning of hybridomas which produce monoclonal antibodies recognizing gp160

In order to maintain the stable subculture of the produced hybridomas, 6-76 and C1-182 cells were cloned. The cells were diluted and seeded in a 96-well tissue culture plate at 0.3,1, and 3 cells per well. After about two weeks, wells in which only a single colony appeared per well were checked under a microscope. Then, their supernatants were collected, and the presence of anti-gp160 antibodies was detected by an ELISA method. In the cloning, a 10% FBS-containing RPMI1640 medium which was further supplemented with 10% BM Condimed H1 (Roche Diagnostics Cat. No.1 088 947) was used. The cloning was carried out twice. Hybridoma strains 6-76-14, 6-76-17, C1-182-40, and C1-182-48 which were positive in the first cloning were used for the second cloning. The positive ratios in the second cloning were respectively 35/36 (97%), 26/26 (100%), 20/29 (69%), and 29/29 (100%), and it was concluded that #6-76 and C1-182 were both successfully cloned (Fig. 10).

Furthermore, Western blotting was performed in the same manner as described in Example 6 using 6-76-14-21, 6-76-14-29, C1-182-48-5, and C1-182-48-35 cells which were positive in the second cloning. As a result, gp160 and gp41 were detected exactly in the same way as that of the original hybridomas and the positive cells in the first cloning (Fig. 11).

### [Example 9] Purification of antibodies produced by hybridoma 6-76-14-29

Antibody protein in the culture supernatant of hybridoma 6-76-14-29 was purified using a column on which immunoglobulin-binding protein L (ImmunoPure Immobilized Protein L, Pierce Chemical Company, Cat. No. 20510) had been immobilized. The protocol was according to the attached instruction. The purified antibody showed a single band each for H chain and L chain in both Sypro Orange fluorescent staining (BioRad Cat. No. 170-3120) and Western blotting using an anti-mouse IgG (L+H) antibody (MP Biomedicals Cat. No. 67428), as in the case of a commercially available anti-ovalbumin antibody (AntibodyShop Cat. No. HYB094-07) used as a control. This result showed that hybridoma 6-76-14-29 was a clone and the produced antibody protein was a single type (Fig. 12). Furthermore, Western blotting was performed using the purified antibody, resulting in the detection of gp160 and gp41 as in the case of the unpurified supernatant (Fig. 13).

### [Example 10] Obtaining of hybridomas recognizing gp120

Immunization, hybridoma production, selection, and Western blotting were performed according to Example 6, except that the immunization was performed by foot pad injection and the vector used for priming was at 2 x 10⁹ CIU/mouse. Multiple hybridomas producing anti-gp160 antibodies which include not only those recognizing gp41 (D1-518, A2-12, B2-39, and B2-103), but also those recognizing gp120 (D1-106, D1-137, D1-526, and D2-26), were successfully obtained (Fig. 14).

### [Example 11] Induction of anti-gp160 antibodies in plasma by administration of an F gene-defective Sendai virus vector simultaneously carrying IL10

The F gene-defective Sendai virus vector SeV18+LacZ/ΔF (WO00/70070) was used to produce a vector simultaneously carrying nucleic acids encoding gp160 and the cytokine interleukin (IL)-10, which is known to have a stimulatory effect on antibody production as well as a suppressive effect on CTL. The resulting vector was used to immunize mice and antibodies produced in the plasma after 56 days were examined. The immunization and Western blotting were performed according to Example 7. It was shown that anti-gp160 antibodies were effectively induced by the Sendai virus vector simultaneously carrying IL-10 (Fig. 15).

### Industrial Applicability

The present invention provides methods for producing antibodies using minus-strand RNA viral vectors. Antibodies produced by the methods of the present invention can be used for detection of protein, purification of protein, neutralization, clinical examination, pathological diagnosis, antibody therapy, and the like.

## Claims

1. A method for producing a monoclonal antibody or a monoclonal antibody-producing cell, which comprises the steps of:
(a) inoculating a non-human animal with a minus-strand RNA viral vector carrying a nucleic acid which encodes a foreign polypeptide to be used as an antigen, a nucleic acid producing the viral vector, a cell into which the vector or the nucleic acid producing the vector has been introduced, wherein the inoculation is performed by palm or foot pad intracutaneous administration;
(b) collecting an antibody-producing cell from the animal;
(c) fusing the collected antibody-producing cell with a myeloma to prepare a hybridoma; and
(d) collecting the hybridoma or a monoclonal antibody produced by the hybridoma.

2. The method of claim 1, further comprising the step of boosting by inoculating with said minus-strand RNA viral vector, said nucleic acid producing the viral vector, said cell into which the vector or the nucleic acid producing the vector has been introduced, said lysate of the cell, or an antigen purified from the lysate of the cell.

3. The method of claim 1, further comprising the step of boosting with said polypeptide or a polypeptide comprising a fragment thereof.

4. The method of claim 2 or 3, wherein the boosting is performed by intraperitoneal administration and/or intracutaneous injection to the dorsum or foot pad.

5. The method of claim 4, wherein the boosting is performed by intraperitoneal administration of said minus-strand RNA viral vector or said lysate of the cell.

6. The method of claim 1, further comprising the step of contacting the collected antibody with said antigen to select an antibody which binds to the antigen.

7. The method of claim 1, further comprising the step of contacting an antibody produced by the hybridoma with said antigen to select a hybridoma producing an antibody that binds to the antigen.

8. The method of claim 1, wherein the minus-strand RNA virus is replication-defective.

9. The method of claim 1, wherein the minus-strand RNA virus is a paramyxovirus.

10. The method of claim 9, wherein the paramyxovirus is at least F gene-defective.

11. The method of claim 9, wherein the paramyxovirus is Sendai virus.

12. The method of claim 1, further comprising the steps of:
(a) allowing a solution comprising the collected antibody to coexist with said minus-strand RNA viral vector that does not encode said foreign polypeptide, a cell into which the viral vector has been introduced, or a viral protein of the virus; and
(b) selecting an antibody that does not bind thereto.

13. The method of claim 1, further comprising the steps of:
(a) allowing a solution comprising an antibody produced by the hybridoma to coexist with a minus-strand RNA viral vector that does not encode said foreign polypeptide, a cell into which such a viral vector has been introduced, or a viral protein of the virus; and
(b) selecting a hybridoma producing an antibody that does not bind thereto.

14. The method of claim 1, further comprising the step of administering a Th2 cytokine or an active partial peptide thereof, or a vector encoding the same.

15. The method of claim 14, wherein the cytokine or active partial peptide thereof is encoded by the minus-strand RNA viral vector which encodes the foreign polypeptide to be used as an antigen.

16. The method of claim 14, wherein the Th2 cytokine is selected from the group consisting of IL-4, IL-10, and IL-13.

## Patentansprüche

1. Verfahren zur Herstellung eines monoclonalen Antikörpers oder einer Zelle, die einen monoclonalen Antikörper herstellt, das die Schritte umfasst:
(a) das Impfen eines nicht-menschlichen Tiers mit einem viralen Minus-Strang-RNA-Vektor, der eine Nucleinsäure trägt, die ein fremdes Polypeptid codiert, das als ein Antigen verwendet werden soll, einer Nucleinsäure, die den viralen Vektor produziert, einer Zelle, in die der Vektor oder die den Vektor produzierende Nucleinsäure eingeführt wurde, wobei die Impfung durch intrakutane Verabreichung in den Hand- oder Fußballen durchgeführt wird;
(b) das Gewinnen einer Zelle aus dem Tier, die einen Antikörper herstellt;
(c) das Fusionieren der gewonnenen Zelle, die einen Antikörper herstellt, mit einem Myelom, um ein Hybridom herzustellen;
(d) das Gewinnen des Hybridoms oder eines monoclonalen Antikörpers, der durch das Hybridom hergestellt wird.

2. Verfahren nach Anspruch 1, ferner umfassend den Schritt des Boosterns durch Impfen mit dem viralen Minus-Strang-RNA-Vektor, der Nucleinsäure, die den viralen Vektor produziert, der Zelle, in die der Vektor oder die den Vektor produzierende Nucleinsäure eingeführt wurde, dem Lysat der Zelle oder einem Antigen, das aus dem Lysat der Zelle gereinigt wurde.

3. Verfahren nach Anspruch 1, ferner umfassend den Schritt des Boosterns mit dem Polypeptid oder einem Polypeptid umfassend ein Fragment davon.

4. Verfahren nach Anspruch 2 oder 3, wobei das Boostern durchgeführt wird durch intraperitoneale Verabreichung und/oder intrakutane Injektion in den Fußrücken oder Fußballen.

5. Verfahren nach Anspruch 4, wobei das Boostern durchgeführt wird durch intraperitoneale Verabreichung des viralen Minus-Strang-RNA-Vektors oder des Lysats der Zelle.

6. Verfahren nach Anspruch 1, ferner umfassend den Schritt des Inkontaktbringens des gewonnenen Antikörpers mit dem Antigen, um einen Antikörper auszuwählen, der an das Antigen bindet.

7. Verfahren nach Anspruch 1, ferner umfassend den Schritt des Inkontaktbringens eines Antikörpers, der von dem Hybridom hergestellt wird, mit dem Antigen, um ein Hybridom auszuwählen, das einen Antikörper herstellt, der an das Antigen bindet.

8. Verfahren nach Anspruch 1, wobei das Minus-Strang-RNA-Virus fehlerhaft in der Replikation ist.

9. Verfahren nach Anspruch 1, wobei das Minus-Strang-RNA-Virus ein Paramyxovirus ist.

10. Verfahren nach Anspruch 9, wobei das Paramyxovirus mindestens im F-Gen fehlerhaft ist.

11. Verfahren nach Anspruch 9, wobei das Paramyxovirus ein Sendai-Virus ist.

12. Verfahren nach Anspruch 1, ferner umfassend die Schritte:
(a) das Ermöglichen des gleichzeitigen Vorhandenseins einer Lösung umfassend den gewonnenen Antikörper mit dem viralen Minus-Strang-RNA-Vektor, der nicht das fremde Polypeptid codiert, einer Zelle, in die der virale Vektor eingeführt wurde, oder einem viralen Protein des Virus; und
(b) das Auswählen eines Antikörpers, der nicht daran bindet.

13. Verfahren nach Anspruch 1, ferner umfassend die Schritte:
(a) das Ermöglichen des gleichzeitigen Vorhandenseins einer Lösung umfassend einen Antikörper, der hergestellt ist von der Hybridomzelle, mit einem viralen Minus-Strang-RNA-Vektor, der nicht das fremde Polypeptid codiert, einer Zelle, in die solch ein viraler Vektor eingeführt wurde, oder einem viralen Protein des Virus; und
(b) das Auswählen eines Hybridoms, das einen Antikörper herstellt, der nicht daran bindet.

14. Verfahren nach Anspruch 1, ferner umfassend den Schritt der Verabreichung eines Th2-Cytokins oder eines aktiven Teilpeptids davon oder eines Vektors, der diese codiert.

15. Verfahren nach Anspruch 14, wobei das Cytokin oder aktive Teilpeptid davon codiert wird durch den viralen Minus-Strang-RNA-Vektor, der das fremde Polypeptid codiert, das als ein Antigen verwendet werden soll.

16. Verfahren nach Anspruch 14, wobei das Th2-Cytokin ausgewählt ist aus der Gruppe bestehend aus IL-4, IL-10 und IL-13.

## Revendications

1. Procédé de production d'un anticorps monoclonal ou d'une cellule productrice d'anticorps monoclonaux, qui comprend les étapes consistant à :
(a) inoculer un animal non humain avec un vecteur viral à ARN à brin négatif portant un acide nucléique qui code pour un polypeptide étranger destiné à être utilisé comme un antigène, un acide nucléique produisant le vecteur viral, une cellule dans laquelle le vecteur ou l'acide nucléique produisant le vecteur a été introduit, dans lequel l'inoculation est réalisée par administration intracutanée dans la paume ou le coussinet de la patte ;
(b) recueillir une cellule productrice d'anticorps chez l'animal ;
(c) fusionner la cellule productrice d'anticorps recueillie avec un myélome pour préparer un hybridome ; et
(d) recueillir l'hybridome ou un anticorps monoclonal produit par l'hybridome.

2. Procédé selon la revendication 1, comprenant, en outre, l'étape de stimulation par inoculation avec ledit vecteur viral à ARN à brin négatif, ledit acide nucléique produisant le vecteur viral, ladite cellule dans laquelle le vecteur ou l'acide nucléique produisant le vecteur a été introduit, ledit lysat cellulaire, ou un antigène purifié à partir du lysat cellulaire.

3. Procédé selon la revendication 1, comprenant, en outre, l'étape de stimulation avec ledit polypeptide ou un polypeptide comprenant un fragment de celui-ci.

4. Procédé selon la revendication 2 ou 3, dans lequel la stimulation est réalisée par administration intra-péritonéale et/ou injection intracutanée au niveau de la partie dorsale ou du coussinet de la patte.

5. Procédé selon la revendication 4, dans lequel la stimulation est réalisée par administration intra-péritonéale dudit vecteur viral à ARN à brin négatif ou dudit lysat cellulaire.

6. Procédé selon la revendication 1, comprenant, en outre, l'étape de mise en contact de l'anticorps recueilli avec ledit antigène afin de sélectionner un anticorps qui se lie à l'antigène.

7. Procédé selon la revendication 1, comprenant, en outre, l'étape de mise en contact d'un anticorps produit par l'hybridome avec ledit antigène afin de sélectionner un hybridome produisant un anticorps qui se lie à l'antigène.

8. Procédé selon la revendication 1, dans lequel le virus à ARN à brin négatif est déficient pour la réplication.

9. Procédé selon la revendication 1, dans lequel le virus à ARN à brin négatif est un paramyxovirus.

10. Procédé selon la revendication 9, dans lequel le paramyxovirus est au moins déficient en gène F.

11. Procédé selon la revendication 9, dans lequel le paramyxovirus est le virus de Sendai.

12. Procédé selon la revendication 1, comprenant, en outre, les étapes consistant à :
(a) permettre à une solution comprenant l'anticorps recueilli de coexister avec ledit vecteur viral à ARN à brin négatif qui ne code pas pour ledit polypeptide étranger, une cellule dans laquelle le vecteur viral a été introduit, ou une protéine virale du virus ; et
(b) sélectionner un anticorps qui ne s'y lie pas.

13. Procédé selon la revendication 1, comprenant, en outre, les étapes consistant à :
(a) permettre à une solution comprenant un anticorps produit par l'hybridome de coexister avec ledit vecteur viral à ARN à brin négatif qui ne code pas pour ledit polypeptide étranger, une cellule dans laquelle un tel vecteur viral a été introduit, ou une protéine virale du virus ; et (b) sélectionner un hybridome produisant un anticorps qui ne s'y lie pas.

14. Procédé selon la revendication 1, comprenant, en outre, l'étape d'administration d'une cytokine Th2 ou d'un peptide partiel actif de celle-ci, ou d'un vecteur codant pour ceux-ci.

15. Procédé selon la revendication 14, dans lequel la cytokine ou un peptide partiel actif de celle-ci est codé par le vecteur viral à ARN à brin négatif qui code pour le polypeptide étranger destiné à être utilisé comme un antigène.

16. Procédé selon la revendication 14, dans lequel la cytokine Th2 est sélectionnée dans le groupe consistant en IL-4, IL-10, et IL-13.
